(19) Europäisches Patentamt
European Patent Office
Office européen des brevets

(11) Veröffentlichungsnummer: 0 257 375 B1

(12) EUROPÄISCHE PATENTSCHRIFT

(45) Veröffentlichungstag der Patentschrift: 28.02.90

(51) Int. Cl.⁴: C07D 249/08 // A01N43/653

(21) Anmeldenummer: 87111250.4

(22) Anmeldetag: 04.08.87

# (54) Verfahren zur Herstellung optisch aktiver 2-Hydroxyethyl-azol- Derivate.

(30) Priorität: 14.08.86 DE 3627673

(43) Veröffentlichungstag der Anmeldung: 02.03.88 Patentblatt 88/9

(45) Bekanntmachung des Hinweises auf die Patenterteilung: 28.02.90 Patentblatt 90/9

(84) Benannte Vertragsstaaten: BE CH DE FR GB IT LI NL

(56) Entgegenhaltungen:
EP-A- 0 040 345
EP-A- 0 052 424
EP-A- 0 085 333
EP-A- 0 108 995
EP-A- 0 135 854
EP-A- 0 181 529

(73) Patentinhaber: BAYER AG, D-5090 Leverkusen 1 Bayerwerk(DE)

(72) Erfinder: Kaulen, Johannes, Dr., Leuchter Gemark 2, D-5060 Berg.-Gladbach(DE)
Erfinder: Arlt, Dieter, Prof. Dr., Rybniker-Strasse 2, D-5000 Köln 80(DE)
Erfinder: Holmwood, Graham, Dr., Krutschelder Weg 105, D-5600 Wuppertal 11(DE)
Erfinder: Krämer, Wolfgang, Dr., Rosenkranz 25, D-5093 Burscheid(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von teilweise bekannten, optisch aktiven 2-Hydroxyethyl-azol-Derivaten, welche fungizide und pflanzenwuchsregulierende Wirksamkeit besitzen.

Es sind bereits Racemate von zahlreichen 2-Hydroxyethyl-azol-Derivaten mit fungiziden und pflanzenwuchsregulierenden Eigenschaften bekannt (vgl. EP-A 0 040 345, EP-A 0 052 424, EP-A 0 084 834 und EP-A 0 111 711). Die Wirkung dieser Stoffe ist aber vor allem bei niedrigen Aufwandmengen nicht immer befriedigend.

Weiterhin ist bekannt, daß sich optisch aktive 2-Hydroxyethyl-azol-Derivate dadurch herstellen lassen, daß man die zugrunde liegenden Racemate von Oxiranen mit optisch aktiven Sulfonsäuren umsetzt, dann das entstehende Diastereomeren-Gemisch in die reinen Diastereomeren auftrennt und anschließend das jeweils gewünschte Diastereomere mit Azol zur Reaktion bringt (vgl. EP-A 181 529 und DE-A 3 440 112). Nachteilig an diesem Verfahren ist jedoch, daß die Ausbeuten an der jeweils gewünschten optisch aktiven Komponente relativ niedrig sind, da bedingt durch die Auftrennung des Diastereomeren-Gemisches zumindest 50% des eingesetzten Materials nicht ausgenutzt werden können.

Es wurde nun gefunden, daß sich optisch aktive 2-Hydroxyethyl-azol-Derivate der Formel

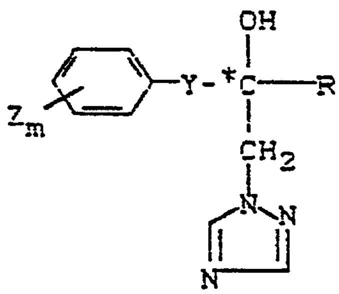

(I)

in welcher

R für gegebenenfalls durch Halogen, Alkoxy mit 1 bis 4 Kohlenstoffatomen, Alkylthio mit 1 bis 4 Kohlenstoffatomen, die CHO-Gruppe und deren Derivate, Phenoxy und/oder Phenyl substituiertes geradkettiges oder verzweigtes Alkyl mit 1 bis 6 Kohlenstoffatomen, für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen und/oder Halogen substituiertes Cycloalkyl mit 3 mit 8 Kohlenstoffatomen oder für gegebenfalls durch Alkyl mit 1 bis 4 Kohlenstoffatomen, Alkoxy mit 1 bis 4 Kohlenstoffatomen, Alkylthio mit 1 bis 4 Kohlenstoffatomen, und/oder Halogen substituiertes Phenyl steht,

Y für die Gruppierungen $-CH_2-CH_2-$, $-CH=CH-$ oder $-CC-$ steht oder für eine direkte Bindung steht,

Z für Fluor, Chlor, Brom, Alkyl mit 1 bis 4 Kohlenstoffatomen, Cycloalkyl mit 3 bis 8 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, gegebenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

herstellen lassen, indem man

a) in einer <u>ersten Stufe</u> Ketone der Formel

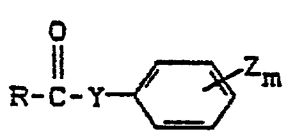

(II)

in welcher

R, Y, Z und m die oben angegebene Bedeutung haben, mit enantiomeren-reinen Oxathianen der Formel

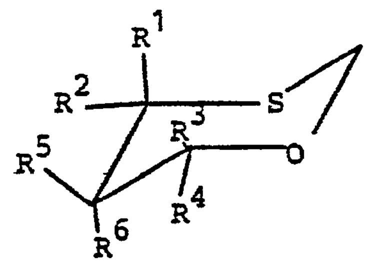

(III)

in welcher

$R^1$, $R^2$, $R^3$, $R^4$, $R^5$, und $R^6$ für Wasserstoff oder Alkyl mit 1 bis 4 Kohlenstoffatomen stehen, wobei jedoch mindestens einer dieser Reste für Alkyl mit 1 bis 4 Kohlenstoffatomen steht,

oder

$R^4$ und $R^5$ auch gemeinsam für gegebenenfalls ein- bis dreifach, gleichartig oder verschieden durch Alkyl mit 1 bis 4 Kohlenstoffatomen substituiertes Alkandiyl mit 3 bis 6 Kohlenstoffatomen stehen oder auch $R^4$ und $R^5$ gemeinsam mit den angrenzenden Kohlenstoffatomen für einen gegebenenfalls ein- bis dreifach, gleichartig oder verschieden durch Alkyl mit 1 bis 4 Kohlenstoffatomen substituierten, anellierten bicyclischen Kohlenwasserstoff mit 7 oder 8 Kohlenstoffatomen stehen, in Gegenwart einer stark basischen metallorganischen Verbindung sowie in Gegenwart eines Verdünnungsmittels bei Temperaturen zwischen –80°C und +120°C umsetzt, dann in einer zweiten Stufe das so erhaltene Gemisch von diastereomeren Verbindungen der Formeln

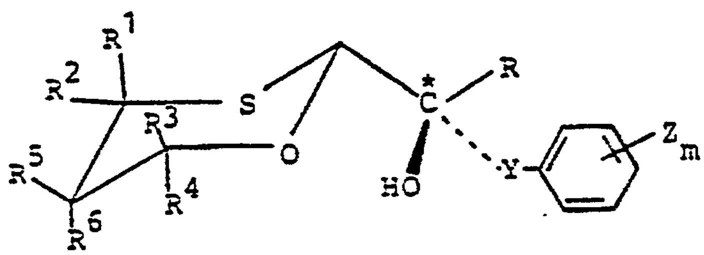

(IV-a)

und

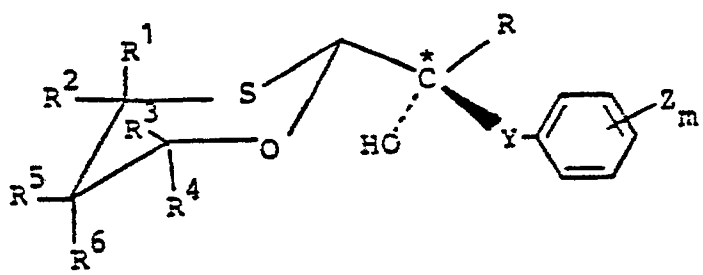

(IV-b)

in welcher

R, $R^1$, $R^2$, $R^3$, $R^4$, $R^5$, $R^6$, Y, Z und m die oben angegebene Bedeutung haben, aufgrund ihrer unterschiedlichen physikalischen Eigenschaften trennt, danach in einer dritten Stufe das jeweils gewünschte Diastereomere der Formel (IV-a) oder (IV-b) zunächst mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0°C und 100°C umsetzt und die dabei entstehenden optisch aktiven α-Hydroxyaldehyde der Formel

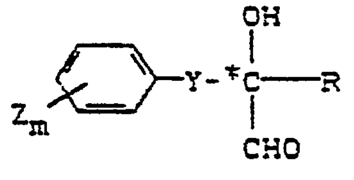

(V)

in welcher

R, Z, Y und mit die oben angegebene Bedeutung haben, mit einem zur Reduktion von Aldehyden geeigneten Reagenz in Gegenwart eines Verdünnungsmittels bei Temperaturen zwischen –20°C und +100°C umsetzt, weiterhin in einer vierten Stufe die so erhaltenen optisch aktiven Diole der Formel

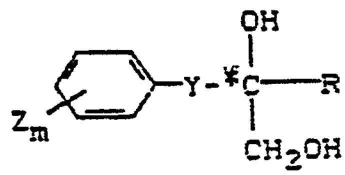

(VI)

in welcher
R, Y, Z und m die oben angegebene Bedeutung haben, mit Sulfonsäure-Derivaten der Formel

$R^7–SO_2–Hal$ (VI)

in welcher
$R^7$ für Alkyl mit 1 bis 4 Kohlenstoffatomen, Halogenalkyl mit 1 oder 2 Kohlenstoffatomen und 1 bis 5 Halogenatomen oder für gegebenenfalls durch Methyl substituiertes Phenyl steht und
Hal für Chlor oder Brom steht,
in Gegenwart eines Verdünnungsmittels und in Gegenwart eines Säurebindemittels bei Temperaturen zwischen 0°C und 100°C umsetzt und schließlich in einer fünften Stufe die entstandenen optisch aktiven Verbindungen der Formel

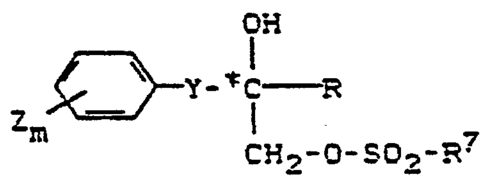

(VIII)

in welcher R, $R^7$, Y, Z und m die oben angegebene Bedeutung haben, mit Triazol-Salzen der Formel

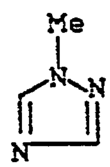

(IX)

in welcher
Me für Natrium oder Kalium steht,
in Gegenwart eines Verdünnungsmittels und gegebenenfalls in Gegenwart eines Säurebindemittels bei Temperaturen zwischen 20°C und 150°C umsetzt,
oder

b) in einer ersten Stufe enantiomerenreine Oxathian-Ketone der Formel

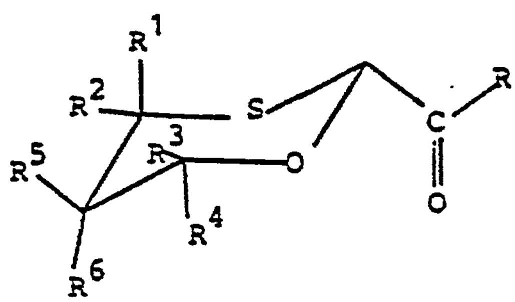

(X)

in welcher
R, $R^1$, $R^2$, $R^3$, $R^4$, $R^5$, und $R^6$ die oben angegebene Bedeutung haben, mit metallorganischen Verbindungen der Formel

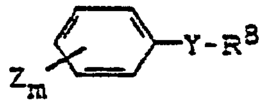

(XI)

in welcher
Y, Z und m die oben angegebene Bedeutung haben und
$R^8$ für Lithium, Natrium, Kalium, Titan-isopropylat, oder einen Rest der Formel $Me^1X$ steht, worin $Me^1$ für Magnesium oder Zink steht und X für Chlor, Brom oder Iod steht,
in Gegenwart eines Verdünnungsmittels bei Temperaturen zwischen –78°C und +100°C umsetzt, danach in einer <u>zweiten Stufe</u> das bevorzugt entstandene Diastereomere der Formel

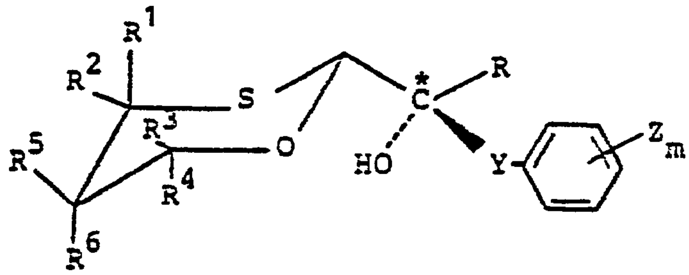

(IV-b)

in welcher
R, $R^1$, $R^2$, $R^3$, $R^4$, $R^5$, $R^6$, Y, Z und m die oben angegebene Bedeutung haben,
gegebenenfalls nach vorheriger chromatoraphischer Abtrennung des in untergeordnetem Maße entstandenen Diastereomeren der Formel (IV-a) zunächst mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0°C und 100°C umsetzt und dann in gleicher Weise wie im Falle der Verfahrensvariante (a) weiterarbeitet,
oder

c) in einer <u>ersten Stufe</u> enantiomeren-reine Oxathian-Ketone der Formel

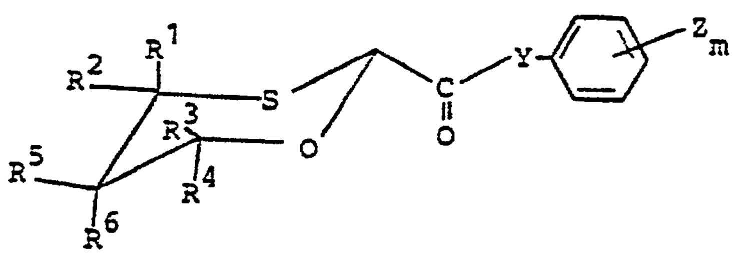

(XII)

in welcher
$R^1$, $R^2$, $R^3$, $R^4$, $R^5$, $R^6$, Y, Z und m die oben angegebene Bedeutung haben, mit metallorganischen Verbindungen der Formel

$R–R^8$ (XIII)

in welcher
R und $R^8$ die oben angegebene Bedeutung haben,
in Gegenwart eines Verdünnungsmittels bei Temperaturen zwischen –78°C und +100°C umsetzt, danach in einer <u>zweiten Stufe</u> das bevorzugt entstandene Diastereomere der Formel

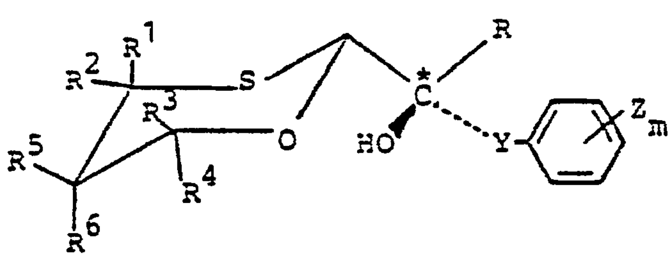

(IV-a)

in welcher

R, $R^1$, $R^2$, $R^3$, $R^4$, $R^5$, $R^6$, Y, Z und m die oben angegebene Bedeutung haben,
gegebenenfalls nach vorheriger chromatograpischer Abtrennung des in untergeordnetem Maße entstandenen Diastereomeren der Formel (IV-b) zunächst mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0°C und 100°C umsetzt und dann in gleicher Weise wie im Falle der Verfahrensvariante (a) weiterarbeitet.

Im vorliegenden Falle wird in den Formelzeichnungen die sterische Anordnung der einzelnen Gruppen nur zum Teil angedeutet. Ferner sind asymmetrisch substituierte Kohlenstoffatome dann durch ein (*) gekennzeichnet, wenn es sich um optisch aktive Verbindungen handelt. Außer den gekennzeichneten Kohlenstoffatomen können noch weitere asymmetrisch substituierte Kohlenstoffatome enthalten sein.

Der Verlauf des erfindungsgemäßen Verfahrens ist als äußerst überraschend zu bezeichnen. So konnte nicht erwartet werden, daß sich optisch aktive 2-Hydroxyethyl-axol-Derivate der Formel (I) nach der erfindungsgemäßen Methode mit hoher Selektivität in sehr guter Ausbeute herstellen lassen. Überraschend ist auch, daß die im Zuge der Umsetzung intermediär anfallenden diastereomeren Verbindungen in einfacher Weise aufgrund ihrer unterschiedlichen physikalischen Eigenschaften getrennt werden können. Schließlich war nicht vorhersehbar, daß nach der erfindungsgemäßen asymmetrischen Totalsynthese sowohl die R- als auch die S-Enantiomeren der Verbindungen der Formel (I) gleichermaßen gut herstellbar sind.

Das erfindungsgemäße Verfahren zeichnet sich durch eine Reihe von Vorteilen aus. So sind die benötigten Ausgangsstoffe und Reaktionskomponenten leicht zugänglich und auch in größeren Mengen verfügbar. Weiterhin bereiten die einzelnen Umsetzungen und die Aufarbeitung der jeweils anfallenden Reaktionsprodukte keinerlei Schwierigkeiten. Hervorzuheben ist außerdem, daß die vor allem im Zuge der Verfahrensvariante (a) durchzuführende Trennung der diastereomeren Verbindungen mit einfachen Mitteln problemlos möglich ist. Besonders günstig ist ferner, daß bei der asymmetrischen Totalsynthese nach den Verfahrensvarianten (b) und (c) das gesamte Ausgangsmaterial in das jeweils gewünschte Enantiomere überführt wird, während bei den vorbekannten Verfahren die Hälfte des eingesetzten Materials nicht ausgenutzt werden kann, da diese Verfahren auf einer Racematspaltung beruhen. Ein entscheidender Vorteil des erfindungsgemäßen Verfahrens besteht schließlich darin, daß sowohl R- als auch S-Enantiomere durch entsprechende Kombination der einzelnen Umsetzungen mit Hilfe des gleichen Hilfsreagenzes herstellbar sind.

Verwendet man bei der Durchführung der Variante (a) des erfindungsgemäßen Verfahrens 2,2-Dimethyl-5-(4-chlorphenyl)-pentan-3-on und enantiomeren-reines Oxathian der Formel (III-1) als Ausgangsstoffe, Kieselgel als stationäre Phase für die chromatographische Trennung in der zweiten Stufe, N-Chlorsuccinimid und Silbernitrat als Reagenz zur Spaltung der Oxathian-Verbindung in der dritten Stufe und Lithiumaluminiumhydrid zur Reduktion des Aldehyds in der dritten Stufe, Chlormethansulfonsäure als Reaktionskomponente in der vierten Stufe und Triazol-Natrium als Reaktionsspartner in der fünften Stufe, so kann der Verlauf des erfindungsgemäßen Verfahrens durch das folgende Formelschema veranschaulicht werden:

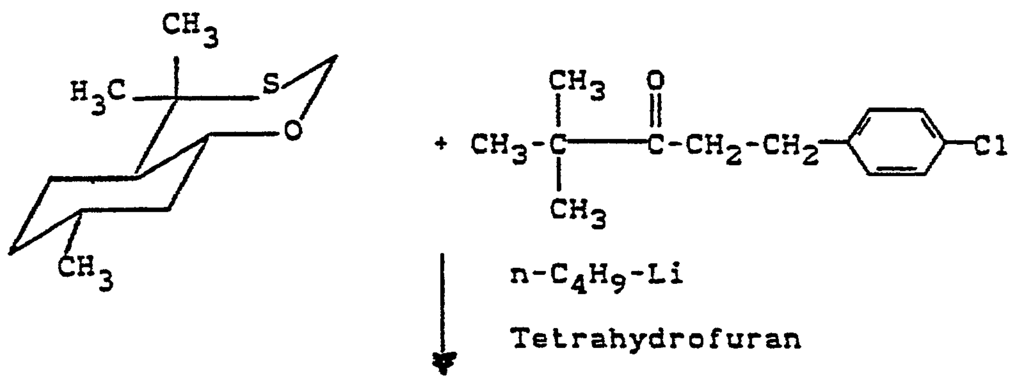


(III-1)

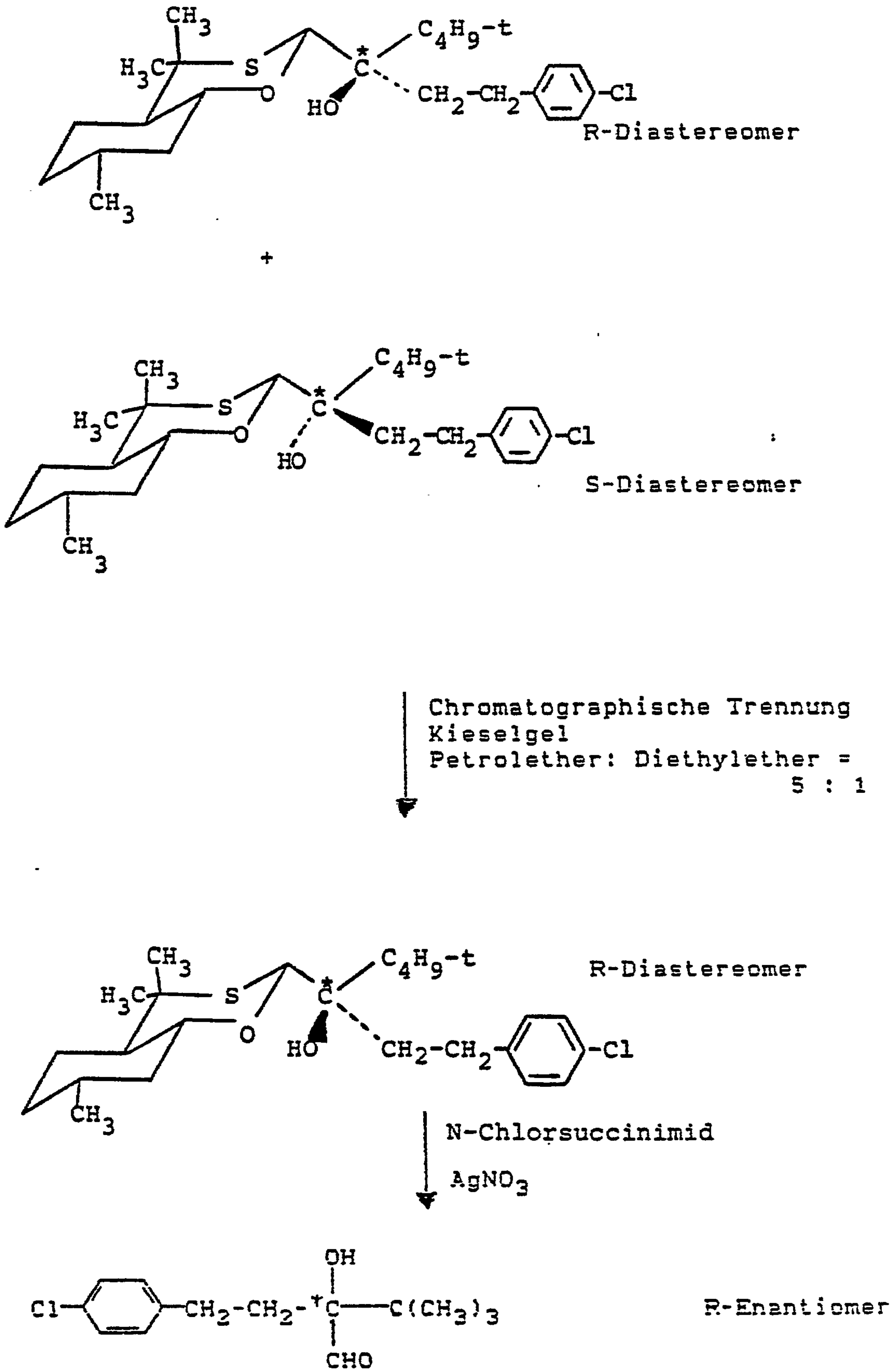


R-Diastereomer

+

S-Diastereomer

R-Diastereomer

R-Enantiomer

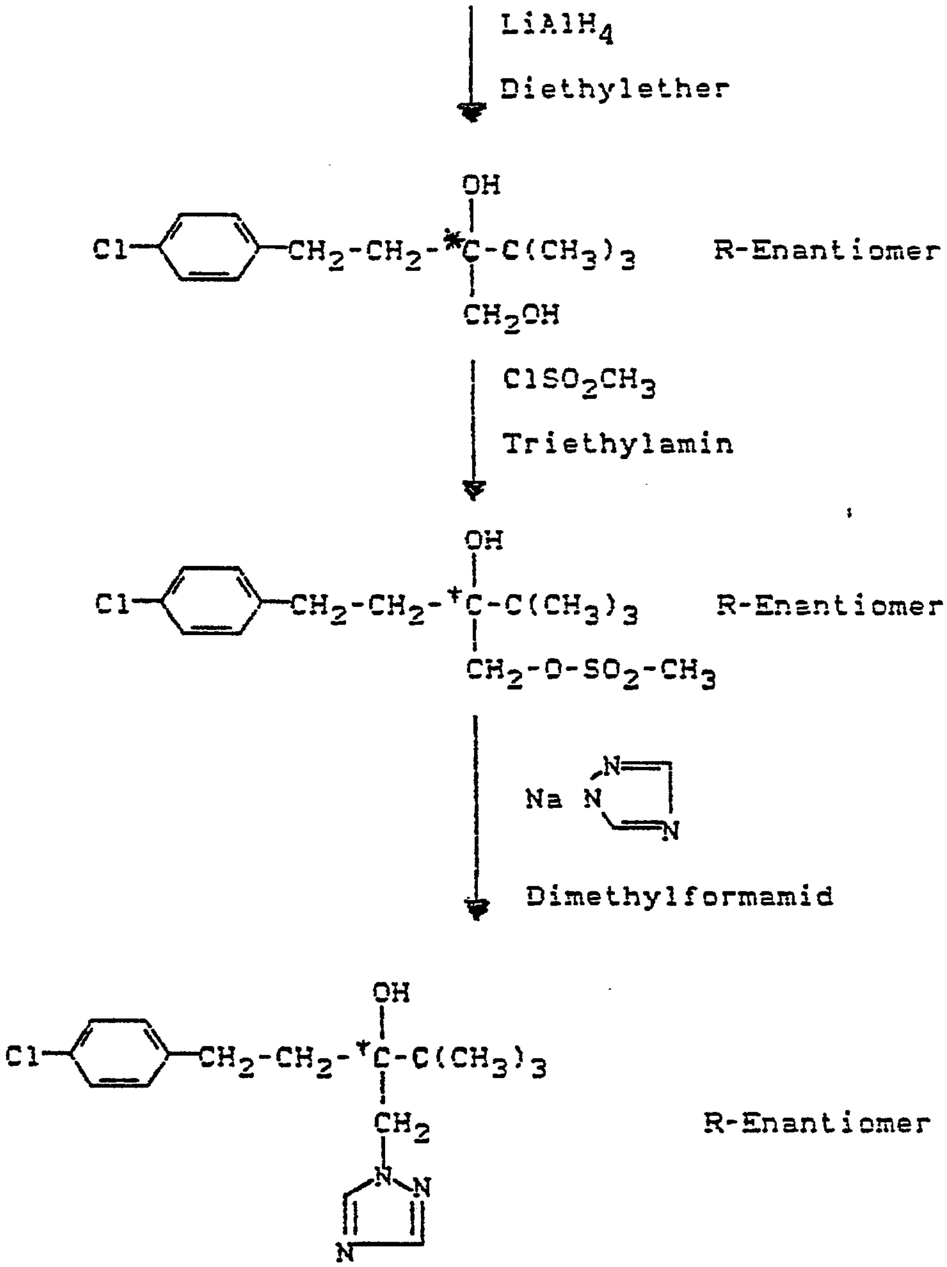


Verwendet man bei der Durchführung der Variante (b) des erfindungsgemäßen Verfahrens vom enantiomeren-reinen Oxathian der Formel (III-1) abgeleitetes tert.-Butylketon und 2-(4-Chlorphenyl)-ethyl-Magnesiumbromid als Ausgangsstoffe, N-Chlorsuccinimid und Silbernitrat als Reagenz zur Spaltung der nahezu ausschließlich entstandenen S-Diastereomeren in der zweiten Stufe und Lithiumaluminiumhydrid zur Reduktion des Aldehyds in der zweiten Stufe, Chlormethansulfonsäure als Reaktionskomponente in der dritten Stufe und Triazol-Natrium als Reaktionspartner in der vierten Stufe, so kann der Verlauf des erfindungsgemäßen Verfahrens durch das folgende Formelschema veranschaulicht werden:

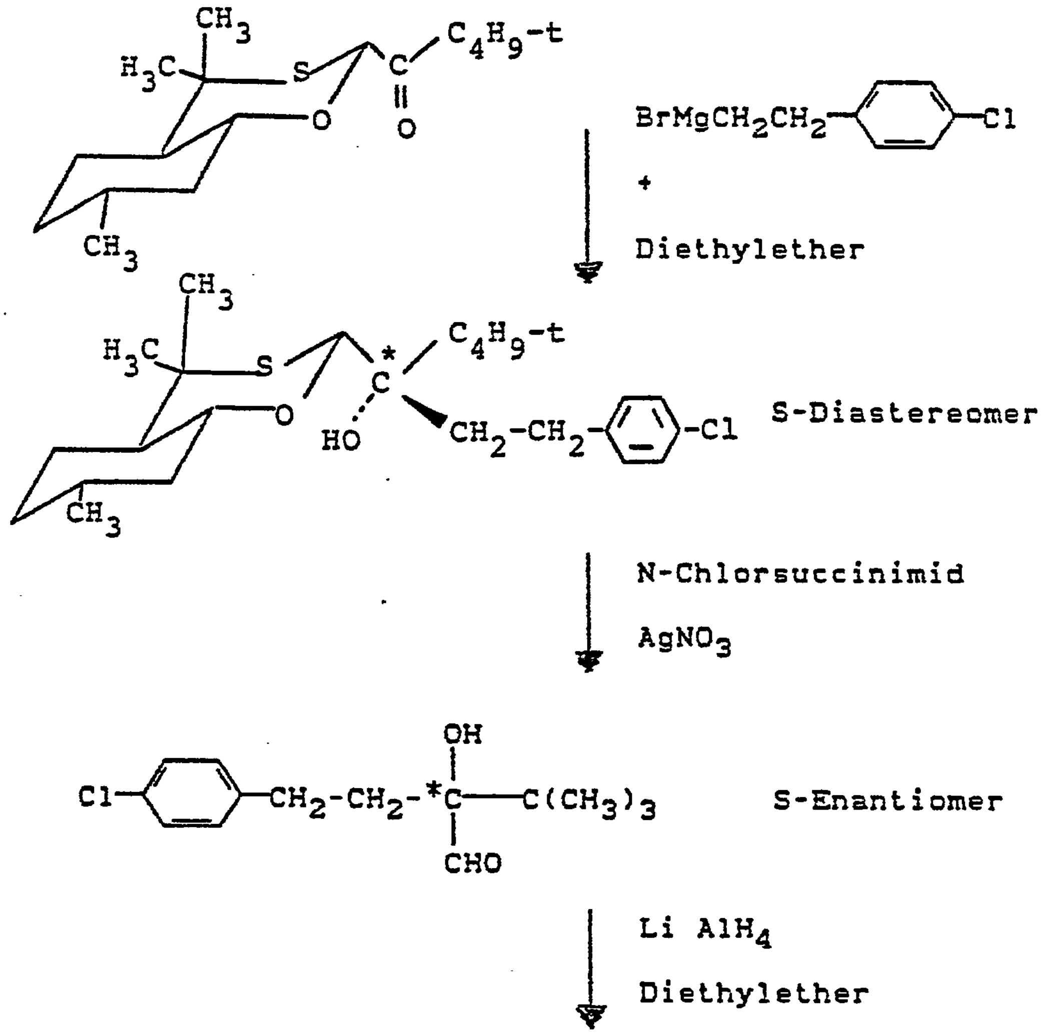
BrMgCH2CH2 Cl
+
Diethylether
S-Diastereomer
N-Chlorsuccinimid
AgNO3
S-Enantiomer
Li AlH4
Diethylether

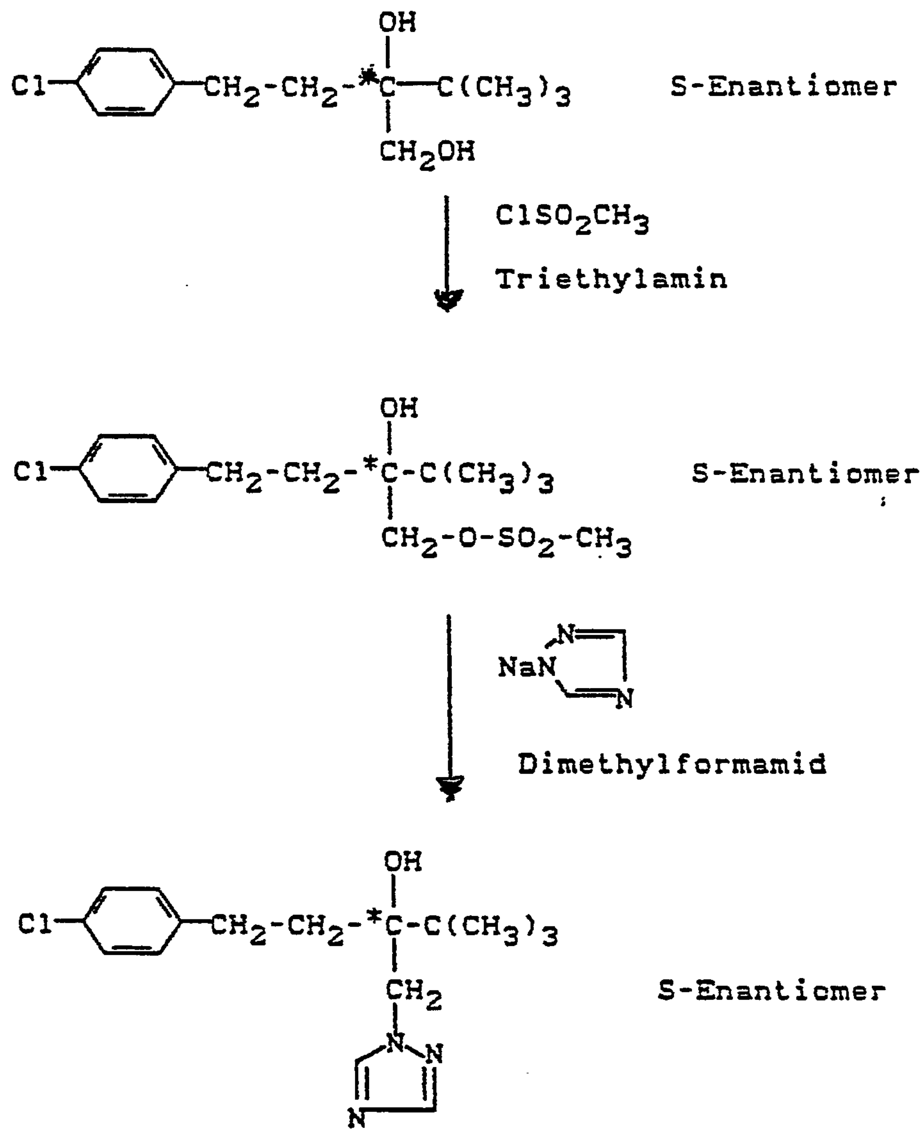


Verwendet man bei der Durchführung der Variante (c) des erfindungsgemäßen Verfahrens vom enantimeren-reinen Oxathian der Formel (III-1) abgeleitetes 2-(4-Chlorpenyl)-ethyl-keton und tert.-Butyl-Magnesiumbromid als Ausgangsstoffe, N-Chlorsuccinimid und Silbernitrat als Reagenz zur Spaltung des nahezu ausschließlich entstandenen R-Diastereomeren in der zweiten Stufe und Lithiumaluminiumhydrid zur Reduktion des Aldehyds in der zweiten Stufe, Chlormethansulfonsäure als Reaktionskomponente in der dritten Stufe und Triazol-Natrium als Reaktionspartner in der vierten Stufe, so kann der Verlauf des erfindungsgemäßen Verfahrens durch das folgende Formelschema veranschaulicht werden:

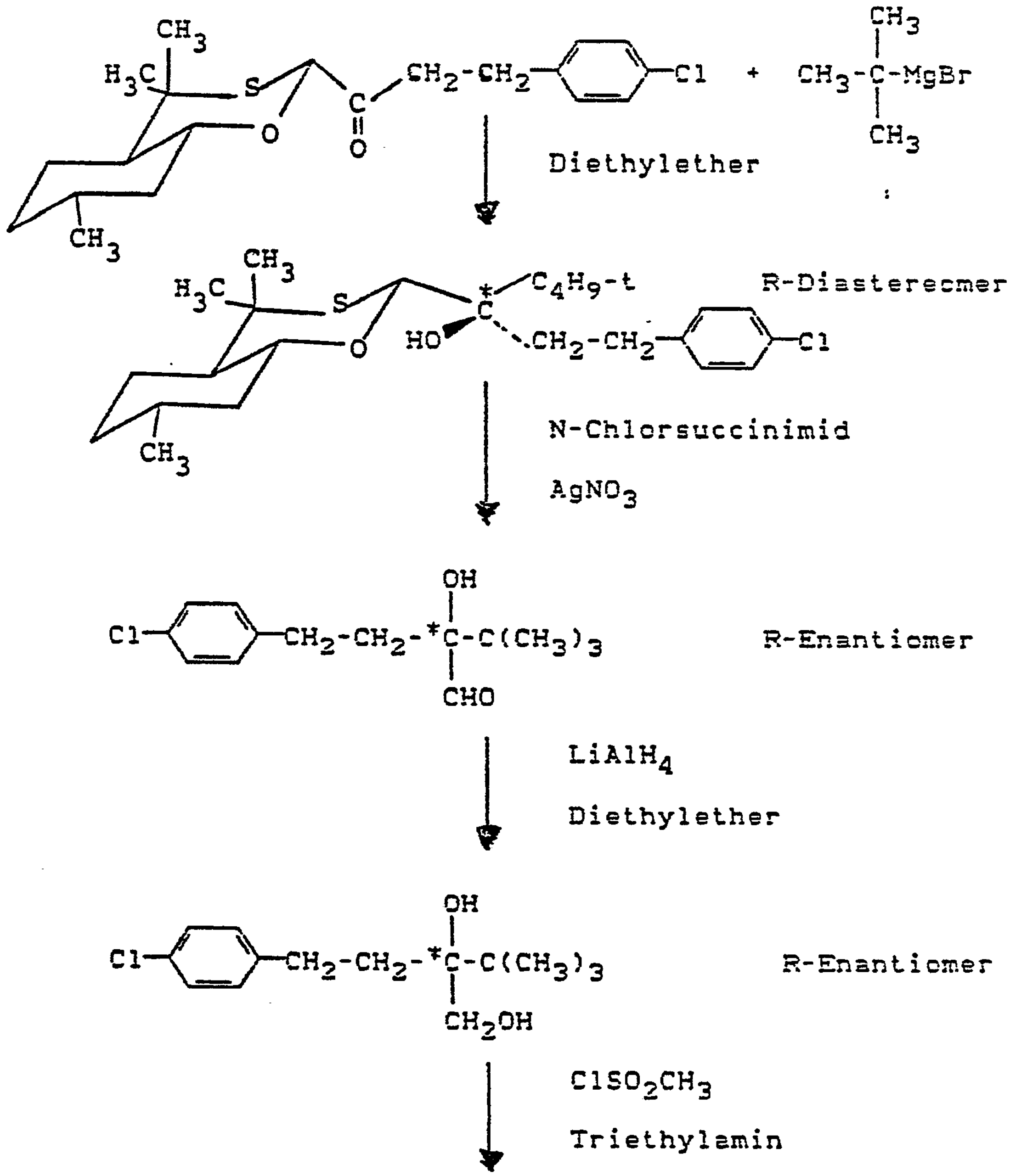
Diethylether
R-Diastereomer
N-Chlorsuccinimid
$AgNO_3$
R-Enantiomer
$LiAlH_4$
Diethylether
R-Enantiomer
$ClSO_2CH_3$
Triethylamin

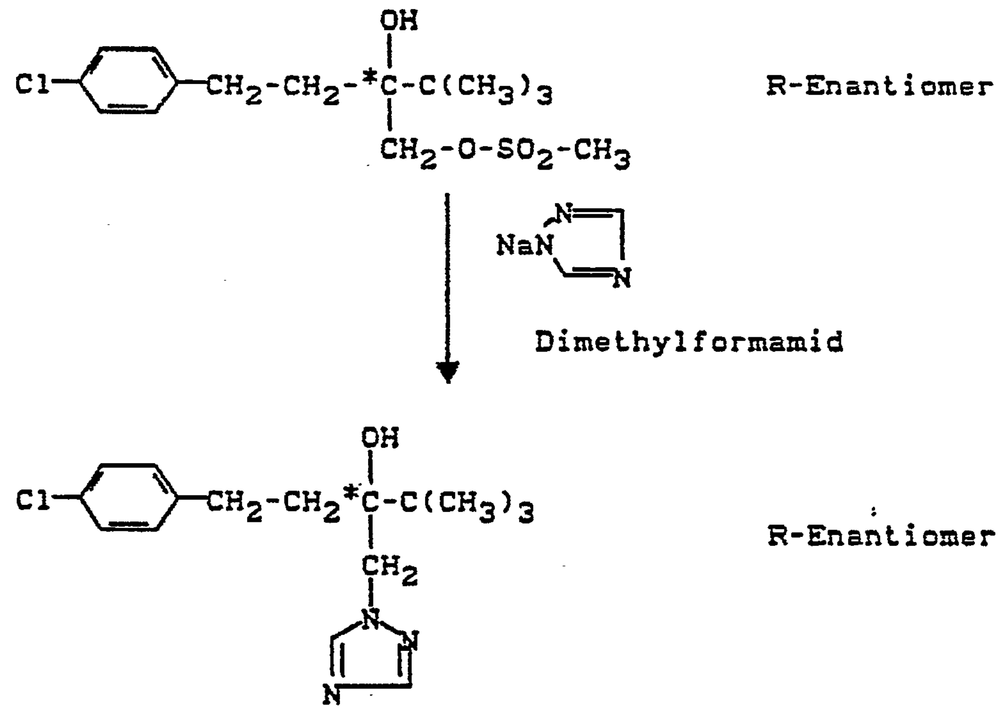


In den obigen Formelzeichnungen und auch im folgenden Teil der Beschreibung bezieht sich die Konfigurationszuordnung "R" bzw. "S" jeweils auf das Carbinol-Kohlenstoffatom.

Die bei der Durchführung des erfindungsgemäßen Verfahrens nach der Variante (a) als Ausgangsstoffe benötigten Ketone sind durch die Formel (II) allgemein definiert.

Besonders bevorzugt sind diejenigen Verbindungen der Formel (II), in denen

R für gegebenenfalls durch Fluor, Chlor, Brom, Alkoxy mit 1 oder 2 Kohlenstoffatomen, Dioxolanyl, Formyl, Methoximinomethyl, Alkylthio mit 1 oder 2 Kohlenstoffatomen, Phenoxy und/oder Phenyl substituiertes Alkyl mit 1 bis 4 Kohlenstoffatomen steht, für gegebenenfalls durch Alkyl mit 1 oder 2 Kohlenstoffatomen, Alkoxy mit 1 oder 2 Kohlenstoffatomen, Alkylthio mit 1 oder 2 Kohlenstoffatomen, Fluor, Chlor und/oder Brom substituiertes Cycloalkyl mit 3 bis 7 Kohlenstoffatomen steht oder für gegebenenfalls durch Alkyl mit 1 oder 2 Kohlenstoffatomen, Alkoxy mit 1 oder 2 Kohlenstoffatomen, Alkylthio mit 1 oder 2 Kohlenstoffatomen, Fluor, Chlor und/oder Brom substituiertes Phenyl steht,

Y für die Gruppierungen $-CH_2-CH_2-$, $-CH=CH-$ oder $-C\equiv C-$ steht oder für eine direkte Bindung steht,

Z für Fluor, Chlor, Brom, Alkyl mit 1 bis 4 Kohlenstoffatomen, Cycloalkyl mit 3 bis 7 Kohlenstoffatomen, Alkoxy mit 1 oder 2 Kohlenstoffatomen, Alkylthio mit 1 oder 2 Kohlenstoffatomen, Halogenmethyl mit 1 bis 3 gleichen oder verschiedenen Halogenatomen, Halogenmethoxy mit 1 bis 3 gleichen oder verschiedenen Halogenatomen, Halogenmethylthio mit 1 bis 3 gleichen oder verschiedenen Halogenatomen, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl oder für gegebenenfalls durch Fluor, Chlor und/oder Methyl substituiertes Benzyloxy steht und

m für die Zahlen 0, 1, 2 oder 3 steht.

Ganz besonders bevorzugt sind diejenigen Stoffe der Formel (II), in denen

R für gegebenenfalls ein- oder zweifach, gleichartig oder verschieden durch Fluor, Chlor, Brom, Methoxy, Methylthio, Methoximinomethyl, Phenoxy und/oder Phenyl substituiertes Methyl, Ethyl, Isopropyl oder tert.-Butyl steht, weiterhin für die Gruppierung

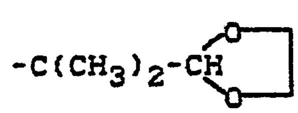

ferner für jeweils gegebenenfalls ein- oder zweifach, gleichartig oder verschieden durch Methyl, Methylthio, Fluor und/oder Chlor substituiertes Cyclopropyl, Cyclopentyl oder Cyclohexyl steht oder für gegebenenfalls ein- oder zweifach, gleichartig oder verschieden durch Methyl, Methoxy, Methylthio, Fluor und/oder Chlor substituiertes Phenyl steht,

Y für die Gruppierungen $-CH_2-CH_2$, $-CH=CH-$, oder $-C\equiv C-$ steht oder für eine direkte Bindung steht,

Y für die Gruppierungen $-CH_2-CH_2$, $-CH=CH-$, oder $-C\equiv C-$ steht oder für eine direkte Bindung steht,
Z für Fluor, Chlor, Brom, Methyl, Ethyl, Isoproyl, tert.-Blutyl, Cyclopropyl, Cyclopentyl, Cyclohexyl, Methoxy, Methylthio, Trifluormethyl, Trifluormethoxy, Trifluormethylthio, gegebenenfalls ein- oder zweifach, gleichartig oder verschieden durch Fluor, Chlor und/oder Methyl substituiertes Phenyl, gegebenenfalls ein- oder zweifach, gleichartig oder verschieden durch Fluor, Chlor und/oder Methyl substituiertes Phenoxy, gegebenenfalls ein- oder zweifach, gleichartig oder verschieden durch Fluor, Chlor und/oder Methyl substituiertes Benzyl oder für gegebenenfalls ein- oder zweifach, gleichartig oder verschieden durch Fluor, Chlor und/oder Methyl substituiertes Benzyloxy steht und
m für die Zahlen 0, 1, 2 oder 3 steht.

Die Ketone der Formel (II) sind bekannt oder lassen sich nach prinzipiell bekannten Methoden in einfacher Weise herstellen (vgl. DE-B 2 201 063, DE-A 2 705 678, DE-A 2 737 489, EP-OS 0 111 711, Tetrahedron 31, 3 (1975) und Chemical Abstracts 84, 73 906 n).

Die bei der Durchführung der Variante (a) des erfindungsgemäßen Verfahrens weiterhin als Ausgangsstoffe benötigten enantiomeren-reinen Oxathiane sind durch die Formel (III) allgemein definiert.

Besonders bevorzugt sind diejenigen Oxathiane der Formel (III), in denen
$R^1$, $R^2$, $R^3$, $R^4$, $R^5$ und $R^6$ für Wasserstoff, Methyl oder Ethyl stehen, wobei jedoch mindestens einer dieser Reste für Methyl oder Ethyl steht, und außerdem
$R^4$ und $R^5$ auch gemeinsam für gegebenenfalls ein- bis dreifach, gleichartig oder verschieden durch Methyl und/oder Ethyl substituiertes Alkandiyl mit 3 bis 5 Kohlenstoffatomen stehen oder auch
$R^4$ und $R^5$ gemeinsam mit den angrenzenden Kohlenstoffatomen für einen gegebenenfalls ein- bis dreifach, gleichartig oder verschieden durch Methyl und/oder Ethyl substituierten, anellierten bicyclischen Kohlenwasserstoff mit 7 oder 8 Kohlenstoffatomen stehen.

Als Beispiele für enantiomeren-reine Oxathiane der Formel (III) seien die Verbindungen der folgenden Formeln genannt:

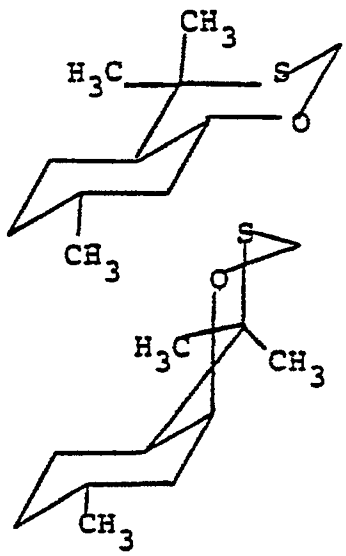

(III-1)

(III-2)

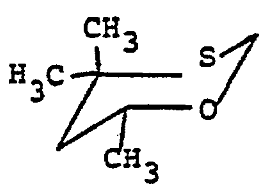

(III-3)

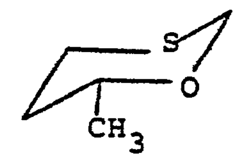

(III-4)

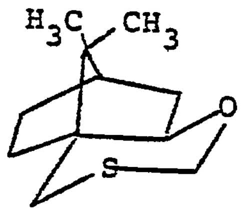

(III-5)

Die enantiomeren-reinen Oxathiane der Formel (III) sind bekannt oder lassen sich nach prinzipiell bekannten Verfahren herstellen (vgl. Phosphorus Sulfur 24 (1985) 4533–475, Asymmetric Synthesis Vol. 2 (1983) 125–155 und Asymmetric Reactions and Processes in Chemistry, ACS Symposium Series No. 185 (1982) 37–53).

Als stark basische metallorganische Verbindungen kommen bei der Durchführung der ersten Stufe der Variante (a) des erfindungsgemäßen Verfahrens alle für derartige Reaktionen üblichen Verbindungen dieses Typs in Betracht. Vorzugsweise verwendbar sind Alkyl-Lithium-Verbindungen, wie n-Butyl-Lithium, ferner Phenyl-Lithium, und außerdem Lithiumamide, wie Lithium-di-isopropylamid.

Als Verdünnungsmittel kommen bei der Durchführung der ersten Stufe der Variante (a) des erfindungsgemäßen Verfahrens alle für derartige Umsetzungen üblichen organischen Solventien in Frage. Vorzugsweise verwendbar sind Ether, wie Diethylether, Tetrahydrofuran und Dioxan, gegebenenfalls unter Zusatz von polaren Solventien, wie Hexamethyl-phosphorsäuretriamid und Tetramethyl-ethylendiamin.

Die Reaktionstemperaturen können bei der Durchführung der ersten Stufe der Variante (a) des erfindungsgemäßen Verfahrens in einem bestimmten Bereich variiert werden. Im allgemeinen arbeitet man bei Temperaturen zwischen –80°C und +120°C, vorzugsweise zwischen –80°C und +100°C.

Bei der Durchführung der ersten Stufe der Variante (a) des erfindungsgemäßen Verfahrens arbeitet man bevorzugt unter Schutzgasatmosphäre, wie zum Beispiel unter Stickstoff oder Argon. Die Reaktionskomponenten der Formeln (II) und (III) werden im allgemeinen im äqivalenten Mengen eingesetzt. Es ist jedoch auch möglich, die eine oder andere Komponente in einem Überschuß zu verwenden. Die Aufarbeitung erfolgt nach üblichen Methoden. Im allgemeinen geht man so vor, daß man das Reaktionsgemisch mit wäßriger Ammoniumchlorid-Lösung versetzt, mehrfach mit einem in Wasser wenig löslichen Solvens extrahiert, die vereinigten organischen Phasen trocknet und einengt.

Die Trennung der diastereomeren Verbindungen kann in der zweiten Stufe der Variante (a) des erfindungsgemäßen Verfahrens nach den für derartige Zwecke geeigneten Methoden vorgenommen werden, also zum Beispiel durch fraktionierte Kristallisation oder mit Hilfe von chromatographischen Verfahren. Im allgemeinen geht man so vor, daß man das in der ersten Stufe erhaltene Produkt ohne vorherige Reinigung über eine Säule chromatographiert. Als stationäre Phase kommt dabei vorzugsweise Kieselgel in Betracht. Als flüssige Phase verwendet man vorzugsweise Gemische aus unpolaren organischen Solventien, wie Gemische aus Kohlenwasserstoffen und Ethern. Beispielsweise genannt seien Gemische aus Petrolether und Diethylether.

Die diastereomeren Verbindungen lassen sich bei der Durchführung de zweiten Sufe der Variante (a) des erfindungsgemäßen Verfahrens aufgund der unterschiedlichen $R_f$-Werte in einfacher Weise trennen. Man isoliert die Verbindungen der Formel (IV-a) bzw. (IV-b), indem man das jeweilige Eluat einengt.

Bei der Durchführung der dritten Stufe der Variante (a) des erfindungsgemäßen Verfahrens kommen als Material zur Spaltung der Oxathian-Verbindungen alle für derartige Zwecke üblichen Substanzen in Frage. Vorzugsweise verwendbar sind Gemische aus N-Chlor-succinimid und Silbernitrat.

Als Verdünnungsmittel kommen bei der Durchführung der Spaltung der Oxathian-Verbindungen alle für derartige Reaktionen üblichen Solventien in Betracht. Vorzugsweise verwendbar sind Gemische aus Wasser und polaren organischen Verdünnungsmitteln, die mit Wasser mischbar sind. Beispielhaft genannt seien Gemische aus Acetonitril und Wasser.

Als Säurebindemittel können bei der Durchführung der Spaltung der Oxathian-Verbindungen alle für derartige Umsetzungen üblichen Säureakzeptoren eingesetzt werden. Vorzugsweise verwendbar sind Alkalimetallcarbonate und Alkalimetallhydrogencarbonate, wie zum Beispiel Natriumhydrogencarbonat.

Die Reaktionstemperaturen können bei der Durchführung der Spaltung der Oxathian-Verbindungen in der dritten Stufe der Variante (a) des erfindungsgemäßen Verfahrens in einem größeren Bereich variiert werden. Im allgemeinen arbeitet man bei Temperaturen zwischen 0°C und 100°C, vorzugsweise zwischen 20°C und 60°C.

Bei der Durchführung der Spaltung der Oxathian-Verbindungen setzt man auf 1 Mol an diastereomerer Verbindung der Formel (IV-a) oder (IV-b) im allgemeinen 1 bis 3 Mol an Spaltungsreagenz sowie einen Überschuß an Säurebindemittel ein. Die Aufarbeitung erfolgt nach üblichen Methoden. Im allgemeinen geht man so vor, daß man das Reaktionsgemisch mit wäßrigen Salzlösungen versetzt, die festen Bestandteile abfiltriert, das Filtrat mehrfach mit in Wasser wenig löslichen organischen Solventien extrahiert, die vereinigten organischen Phasen wäscht, trocknet und einengt. Das anfallende Produkt kann ohne zusätzliche Reinigung für die weiteren Umsetzungen verwendet werden.

Bei der Durchführung der Variante (a) des erfindungsgemäßen Verfahrens kommen als Reagenzien zur Reduktion von optisch aktiven α-Hydroxyaldehyden der Formel (V) vorzugsweise komplexe Hydride, wie Lithiumaluminiumhydrid und Natriumborhydrid, in Betracht.

Als Verdünnungsmittel kommen bei der Reduktion von optisch aktiven α-Hydroxyaldehyden der Formel (V) alle inerten organischen Solventien in Frage. Vorzugsweise verwendbar sind Ether, wie Diethylether oder Tetrahydrofuran. Arbeitet man mit Natriumborhydrid als Reduktionsmittel, so ist es auch möglich, Alkohole, wie Methanol oder Ethanol, oder Nitrile, wie Acetonitril, gegebenenfalls im Gemisch mit Wasser, als Verdünnungsmittel einzusetzen.

Die Reaktionstemperaturen können bei der Durchführung der Reduktion in der dritten Stufe der Variante (a) des erfindungsgemäßen Verfahrens innerhalb eines bestimmten Bereichs variiert werden. Im allgemeinen arbeitet man bei Temperaturen zwischen –20°C und +100°C, vorzugsweise zwischen 20°C und 60°C.

Bei der Durchführung der Reduktion in der dritten Stufe der Variante (a) des erfindungsgemäßen Verfahrens setzt man auf 1 Mol an optisch aktiven α-Hydroxyaldehyd der Formel (V) im allgemeinen 1 bis 5 Mol an Reduktionsmittel ein. Die Aufarbeitung erfolgt nach üblichen Methoden. Im allgemeinen geht man in der Weise vor, daß man das Reaktionsgemisch mit einem in Wasser wenig löslichen organischen Solvens sowie mit wäßriger Alkalilauge versetzt, die vorhandenen festen Bestandteile absaugt und die organische Phase nach dem Trocknen einengt. Es ist jedoch auch möglich, das Reaktionsgemisch nach beendeter Umsetzung zunächst einzuengen, das verbleibende Produkt mit einem in Wasser wenig löslichen organischen Solvens zu extrahieren, die vereinigten organischen Phasen zu trocknen und anschließend einzuengen. Das anfallende Produkt kann, gegebenenfalls nach vorheriger Reinigung, für die weiteren Umsetzungen verwendet werden.

Die bei der Durchführung der vierten Stufe der Variante (a) des erfindungsgemäßen Verfahrens als Reaktionskomponenten benötigen Sulfonsäure-Derivate sind durch die Formel (VII) allgemein definiert.

Als Beispiele für Sulfonsäure-Derivate der Formel (VII) seien genannt: Methansulfonsäurechlorid, Ethansulfonsäurechlorid, Trifluormethan-sulfonsäurechlorid und p-Tolylsulfonsäurechlorid.

Die Sulfonsäure-Derivate der Formel (VII) sind allgemein bekannte Verbindungen der organischen Chemie.

Als Säurebindemittel kommen bei der Durchführung der vierten Stufe der Variante (a) des erfindungsgemäßen Verfahrens vorzugsweise niedere tertiäre Alkylamine, Cycloalkylamine, Aralkylamine oder Arylamine in Betracht. Als Beispiele genannt seien Triethylamin, N,N-Dimethyl-benzylamin, Pyridin, 1,4-Diazabicyclo[2,2,2]octan und 1,5-Diazabicyclo[4,3,0]non-5-en.

Als Verdünnungsmittel kommen bei der Durchführung der vierten Stufe der Variante (a) des erfindungsgemäßen Verfahrens alle für derartige Umsetzungen üblichen organischen Solventien in Frage. Vorzugsweise verwendbar sind halogenierte Kohlenwasserstoffe, wie Methylenchlorid, Chloroform und Tetrachlorkohlenstoff.

Die Reaktionstemperaturen können bei der Durchführung der vierten Sufe der Variante (a) des erfindungsgemäßen Verfahrens innerhalb eines bestimmten Bereiches variiert werden. Im allgemeinen arbeitet man bei Temperaturen zwischen 0°C und 100°C, vorzugsweise zwischen 0°C und 50°C.

Bei der Durchführung der vierten Stufe der Variante (a) des erfindungsgemäßen Verfahrens setzt man auf 1 Mol an optisch aktivem Diol der Formel (IV) im allgemeinen 1 bis 1,3 Mol an Sulfonsäure-Derivat der Formel (VII) sowie 1 bis 1,3 Mol an Säurebindemittel ein. Die Aufarbeitung erfolgt nach üblichen Methoden. Im allgemeinen geht man so vor, daß man das Reaktionsgemisch zunächst mit schwach saurer und danach mit schwach basischer wäßriger Lösung wäscht, dann trocknet und einengt. Das anfallende Produkt kann ohne zusätzliche Reinigung dür die weitere Umsetzung verwendet werden.

Die bei der Durchführung der fünften Stufe der Variante (a) des erfindungsgemäßen Verfahrens als Reaktionskomponenten benötigten Triazol-Salze sind durch die Formel (IX) definiert.

Die Triazol-Salze der Formel (IX) sind bekannt.

Als Säurebindemittel können bei der Durchführung der fünften Stufe der Variante (a) des erfindungsgemäßen Verfahrens alle üblichen organischen und anorganischen Basen eingesetzt werden. Vorzugsweise verwendbar sind Alkalicarbonate, wie beispielsweise Natriumcarbonat oder Natriumhydrogencarbonat, ferner niedere tertiäre Alkylamine, Cycloalkylamine, Arylalkylamine oder Arylamine, wie beispielsweise Triethylamin, N,N-Dimethylbenzylamin, Pyridin, 1,4-Diazabicyclo[2,2,2]-octan oder 1,5-Diazabicyclo-[4,3,0]-non-5-en.

Als Verdünnungsmittel kommen bei der Durchführung der fünften Stufe der Variante (a) des erfindungsgemäßen Verfahrens alle für derartige Umsetzungen üblichen inerten organischen Solventien in Frage. Vorzugsweise verwenbar sind polare, aprotische Verdünnungsmittel, wie Dimethylformamid und Dimethylsulfoxid.

Die Reaktionstemperaturen können bei der Durchführung der fünften Stufe der Variante (a) des erfindungsgemäßen Verfahrens in einem größeren Bereich variiert werden. Im allgemeinen arbeitet man bei Temperaturen zwischen 20°C und 150°C, vorzugsweise zwischen 50°C und 150°C.

Die Umsetzungen in der fünften Stufe der Variante (a) des erfindungsgemäßen Verfahrens werden vorzugsweise unter Schutzgasatmosphäre, wie zum Beispiel unter Stickstoff oder Argon, durchgeführt.

Bei der Durchführung der fünften Stufe der Variante (a) des erfindungsgemäßen Verfahrens setzt man auf 1 Mol an optisch aktiver Verbindung der Formel (VIII) im allgemeinen 2 bis 4 Mol an Triazol-Salz der Formel (IX) ein. Die Aufarbeitung erfolgt nach üblichen Methoden. Im allgemeinen geht man so vor, daß man das Reaktionsgemisch, gegebenenfalls nach vorherigem Abziehen des Verdünnungsmittels, mit Wasser sowie mit einem in Wasser wenig löslichen organischen Solvens versetzt, die organische Phase abtrennt, trocknet und einengt und den verbleibenden Rückstand auf chromatograpischem Wege reinigt.

Die bei der Durchführung der ersten Stufe der Variante (b) des erfindungsgemäßen Verfahrens als Ausgangsstoffe benötigten Oxathianketone sind durch die Formel (X) allgemein definiert. In dieser Formel haben R, $R^1$, $R^2$, $R^3$, $R^4$, $R^5$, und $R^6$ diejenigen Bedeutungen, die bereits im Zusammenhang mit der Beschreibung der Verbindungen der Formeln (II) und (III) für diese Reste genannt wurden.

Die Oxathian-Ketone der Formel (X) sind teilweise bekannt. Sie lassen sich herstellen, indem man enantiomeren-reine Oxathiane der Formel

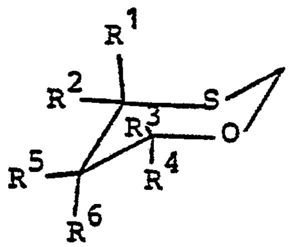

(III)

in welcher

$R^1$, $R^2$, $R^3$, $R^4$, $R^5$ und $R^6$ die oben angegebene Bedeutung haben

mit Aldehyden der Formel

R–CHO (XIV)

in welcher

R die oben angegebene Bedeutung hat,

in Gegenwart einer stark basischen metall-organischen Verbindung, wie n-Butyl-lithium oder Phenyllithium, in Gegenwart eines Verdünnungsmittels, wie Diethylether oder Tetrahydrofuran, unter Schutzgasatmosphäre bei Temperaturen zwischen –80°C und +120°C umsetzt und die entstehenden Carbinole der Formel

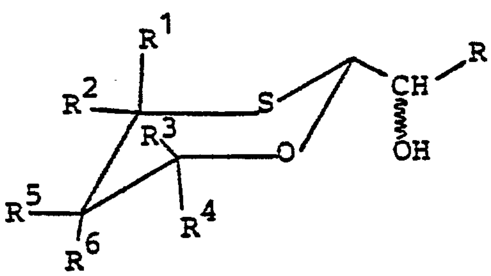

(XV)

in welcher

R, $R^1$, $R^2$, $R^3$, $R^4$, $R^5$ und $R^6$ die oben angegebene Bedeutung haben,

oxidiert. Das bevorzugte Oxidationsverfahren besteht in der Umsetzung mit Dimethylsulfoxid in Gegenwart von Trifluoracetanhydrid und Triethylamin und in Gegenwart eines zusätzlichen Verdünnungsmittels, wie Methylenchlorid, bei Temperaturen zwischen –80°C und +50°C.

Die bei der Durchführung der Variante (b) des erfindungsgemäßen Verfahrens in der ersten Stufe als Reaktionskomponenten benötigten metallorganischen Verbindungen sind durch die Formel (XI) allgemein definiert.

Die metallorganischen Verbindungen der Formel (XI) sind bekannt oder lassen sich nach prinzipiell bekannten Verfahren herstellen.

Als Verdünnungsmittel kommen bei der Durchführung der ersten Stufe der Variante (b) des erfindungsgemäßen Verfahrens alle für derartige Umsetzungen üblichen organischen Solventien in Fragen. Vorzugsweise verwendbar sind Ether, wie Diethylether, Tetrahydrofuran und Dioxan.

Die Reaktionstemperaturen können bei der Durchführung der ersten Stufe der Variante (b) des erfindungsgemäßen Verfahrens in einem bestimmten Bereich variiert werden. Im allgemeinen arbeitet man bei Temperaturen zwischen –78°C und +100°C, vorzugsweise zwischen –78°C und +90°C.

Bei der Durchführung der ersten Stufe der Variante (b) des erfindungsgemäßen Verfahrens setzt man auf 1 Mol an Oxathian-Keton der Formel (X) im allgemeinen 1 bis 2 Mol an metallorganischer Verbindung der Formel (XI) ein. Die Aufarbeitung erfolgt nach üblichen Methoden. Im allgemeinen arbeitet man in der Weise, daß man das Reaktionsgemisch mit wäßriger Ammoniumchlorid-Lösung versetzt, die organische Phase abtrennt, die wäßrige Phase mehrfach mit einem mit Wasser wenig mischbaren organischen Solvens extrahiert, die vereinigten organischen Phasen, trocknet, einengt und unter vermindertem Druck andestilliert. Eine eventuell erforderliche weitere Reinigung kann nach üblichen Methoden durchgeführt werden.

Das in der ersten Stufe der Variante (b) des erfindungsgemäßen Verfahrens erhaltene Produkt besteht zu mehr als 95% aus einem der beiden möglichen Diastereomeren der Formel (IV). Im allgemeinen ist es nicht erforderlich, das in geringer Menge vorhandene Diastereomere vor der weiteren Umsetzung abzutrennen. Die Abtrennung kann jedoch in einfacher Weise erfolgen. Man verfährt dabei so, wie es für die zweite Stufe der Variante (a) des erfindungsgemäßen Verfahrens beschrieben wurde.

Bei der Durchführung der dritten Stufe der Variante (b) des erfindungsgemäßen Verfahrens kommen als Materialien zur Spaltung der Oxathian-Verbindungen alle für derartige Zwecke üblichen Substanzen in Frage. Vorzugsweise verwendbar sind Gemische aus N-Chlor-succinimid und Silbernitrat.

Im übrigen entsprechen die Umsetzungsbedingungen bei der Durchführung der zweiten Stufe der Variante (b) des erfindungsgemäßen Verfahrens denjenigen, die in der dritten Stufe der Variante (a) des erfindungsgemäßen Verfahrens angewandt werden. Auch die weiteren Umsetzungen bei der Durchführung der Variante (b) des erfindungsgemäßen Verfahrens erfolgen in der Weise, wie es im Zusammenhang mit der Variante (a) beschrieben wurde.

Die bei der Durchführung der Variante (c) des erfindungsgemäßen Verfahrens als Ausgangsstoffe benötigten Oxathian-Ketone sind durch die Formel (XII) allgemein definiert. In dieser Formel haben $R^1$, $R^2$, $R^3$, $R^4$, $R^5$, $R^6$, Y, Z und m diejenigen Bedeutungen, die bereits im Zusammenhang mit der Beschreibung der Verbindungen der Formeln (II) und (III) für diese Reste und den Index m genannt wurden.

Die Oxathian-Ketone der Formel (XII) lassen sich herstellen, indem man enantiomeren-reine Oxathiane der Formel

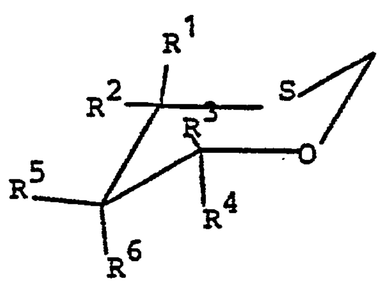

(III)

in welcher

$R^1$, $R^2$, $R^3$, $R^4$, $R^5$ und $R^6$ die oben angegebene Bedeutung haben,

mit Aldehyden der Formel

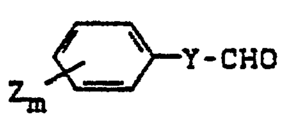

(XVI)

in welcher

Y, Z und m die oben angegebene Bedeutung haben,

in Gegenwart einer stark basischen metallorganischen Verbindung, wie n-Butyl-lithium oder Phenyllithium, in Gegenwart eines Verdünnungsmittels, wie Diethylether oder Tetrahydrofuran, unter Schutzgasatmosphäre bei Temperaturen zwischen –80°C und +120°C umsetzt und die entstehenden Carbinole der Formel

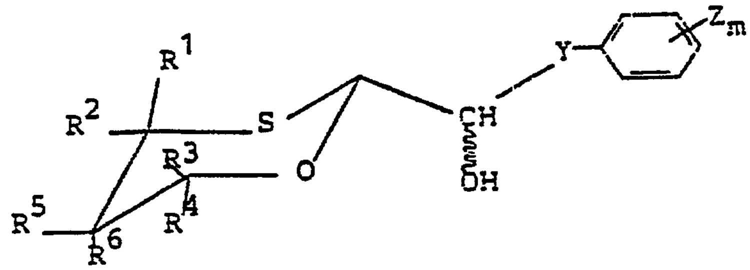

(XVII)

in welcher
$R^1$, $R^2$, $R^3$, $R^4$, $R^5$, $R^6$, Y, Z und m die oben angegebene Bedeutung haben,
mit Dimethylsulfoxid in Gegenwart von Trifluoracetanhydrid und Triethylamin und in Gegenwart eines zusätzlichen Verdünnungsmittels, wie Methylenchlorid, bei Temperaturen zwischen –80°C und +50°C umsetzt.

Die bei der Durchführung der Variante (c) des erfindungsgemäßen Verfahrens in der ersten Stufe als Reaktionskomponenten benötigen metallorganischen Verbindungen sind durch die Formel (XIII) allgemein definiert. Bevorzugt sind diejenigen Verbindungen der Formel (XIII), in denen R und $R^8$ diejenigen Bedeutungen haben, die bereits im Zusammenhang mit der Beschreibung der Verbindungen der Formeln (II) und (XI) als bevorzugt für diese Reste genannt wurden.

Die metallorganischen Verbindungen der Formel (XIII) sind bekannt oder lassen sich nach prinzipiell bekannten Verfahren herstellen.

Als Verdünnungsmittel kommen bei der Durchführung der ersten Stufe der Variante (c) des erfindungsgemäßen Verfahrens alle für derartige Umsetzungen üblichen organischen Solventien in Frage. Vorzugsweise verwendbar sind Ether, wie Diethylether, Tetrahydrofuran und Dioxan.

Die Reaktionstemperaturen können bei der Durchführung der ersten Stufe der Variante (c) des erfindungsgemäßen Verfahrens in einem bestimmten Bereich variiert werden. Im allgemeinen arbeitet man bei Temperaturen zwischen –78°C und +100°C, vorzugsweise zwischen –78°C und +90°C.

Bei der Durchführung der ersten Stufe der Variante (c) des erfindungsgemäßen Verfahrens setzt man auf 1 Mol an Oxathian-Keton der Formel (XIII) im allgemeinen 1 bis 2 Mol an metallorganischer Verbindung der Formel (XIII) ein. Die Aufarbeitung erfolgt nach üblichen Methoden. Im allgemeinen arbeitet man in der Weise, daß man das Reaktionsgemisch mit wäßriger Ammoniumchlorid-Lösung versetzt, die organische Phase abtrennt, die wäßrige Phase mehrfach mit einem mit Wasser wenig mischbaren organischen Solvens extrahiert, die vereinigten organischen Phasen, trocknet, einengt und unter vermindertem Druck andestilliert. Eine eventuell erforderliche weitere Reinigung kann nach üblichen Methoden durchgeführt werden.

Das in der ersten Stufe der Variante (c) des erfindungsgemäßen Verfahrens erhaltene Produkt besteht zu mehr als 95% aus einem der beiden möglichen Diastereomeren der Formel (IV). Im allgemeinen ist es nicht erforderlich, das in geringer Menge vorhandene Diastereomere vor der weiteren Umsetzung abzutrennen. Die Abtrennung kann jedoch in einfacher Weise auf chromatographischem Wege erfolgen. Man verfährt dabei so, wie es für die zweite Stufe der Variante (a) des erfindungsgemäßen Verfahrens beschrieben wurde.

Bei der Durchführung der dritten Stufe der Variante (c) des erfindungsgemäßen Verfahrens kommen als Materialien zur Spaltung der Oxathian-Verbindungen alle für derartige Zwecke üblichen Substanzen in Frage. Vorzugsweise verwendbar sind Gemische aus N-Chlor-succinimid und Silbernitrat.

Im übrigen entsprechen die Umsetzungsbedingungen bei der Durchführung der zweiten Stufe der Variante (c) des erfindungsgemäßen Verfahrens denjenigen, die in der dritten Stufe der Variante (a) des erfindungsgemäßen Verfahrens angewandt werden. Auch die weiteren Umsetzungen bei der Durchführung der Variante (c) des erfindungsgemäßen Verfahrens erfolgen in der Weise, wie es im Zusammenhang mit der Variante (a) beschrieben wurde.

Die Variante (a) des erfindungsgemäßen Verfahrens eignet sich sowohl zur Herstellung derjenigen optisch aktiven 2-Hydroxyethyl-axol-Derivate der Formel (I), die am asymmetrisch substituierten Kohlenstoffatom die R-Konfiguration aufweisen, als auch zur Synthese der entsprechenden S-Enantiomeren. Die Varianten (b) und (c) sind besonders geeignet, um entweder die R- oder die S-Enantiomeren herzustellen. Erhält man bei der Synthese einer bestimmten Verbindung nach der Variante (b) das R-Enantiomere, so fällt bei der Herstellung der gleichen Verbindung nach der Variante (c) das entsprechende S-Enantiomere an. Mit Hilfe des erfindungsgemäßen Verfahrens lassen sich also gezielt die jeweils gewünschten Enantiomeren der 2-Hydroxyethyl-azol-Derivate der Formel (I) herstellen.

Die optisch aktiven 2-Hydroxyethyl-azol-Derivate der Formel

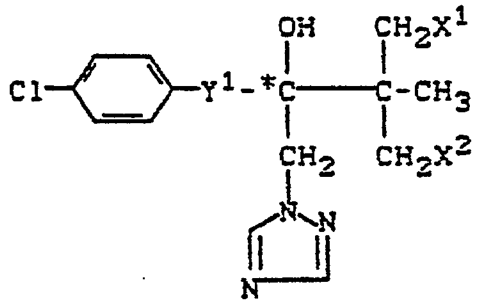

(Ia)

in welcher
$X^1$ für Wasserstoff, Fluor oder Chlor steht,
$X^2$ für Wasserstoff, Fluor oder Chlor steht und
$Y^1$ für die Gruppierungen $-CH_2-CH_2-$, $-CH=CH-$ oder $-C\equiv C-$ steht,
sind zum Teil neu.

Die nach dem erfindungsgemäßen Verfahren herstellbaren optisch aktiven 2-Hydroxyethyl-azol-Derivate der Formel (I) zeichnen sich durch hervorragende fungizide und pflanzenwuchsregulierende Eigenschaften aus. Sie übertreffen die entsprechenden Racemate jeweils in ihrer Wirkung.

Die erfindungsgemäß herstellbaren Wirkstoffe weisen eine starke mikrobizide Wirkung auf und können als Fungizide eingesetzt werden.

Fungizide werden Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der erfindungsgemäß herstellbaren Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Außerdem besitzen die erfindungsgemäß herstellbaren Wirkstoffe auch pflanzenwuchsregulierende Eigenschaften.

Die erfindungsgemäß herstellbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab vom dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist vom großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen könnnen in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Diese ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäß herstellbaren Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommmen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethyformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoff, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäß herstellbaren Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die erfindungsgemäß herstellbaren Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubmittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäß herstellbaren Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-% vorzugsweise von 0,0001 bis 0,02%, am Wirkungsort erforderlich.

Beim Einsatz der erfindungsgemäß herstellbaren Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäß herstellbaren Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Durchführung des erfindungsgemäßen Verfahrens wird durch die folgenden Beispiele veranschaulicht.

Beispiel 1

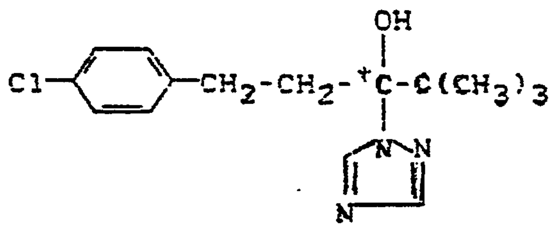

(I-1)

R-Enantiomer

Verfahrensvariante (a)

1. Stufe

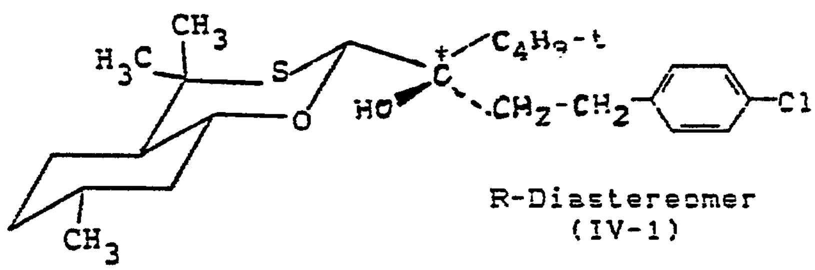

R-Diastereomer
(IV-1)

und

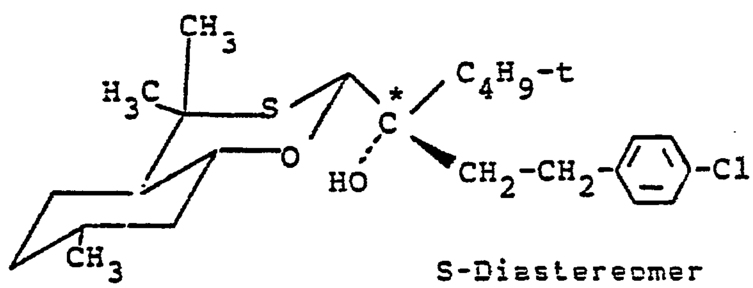

S-Diastereomer
(IV-2)

Zu einer Lösung von 18,0 g (90 mMol) an Oxathian-Verbindung der Formel

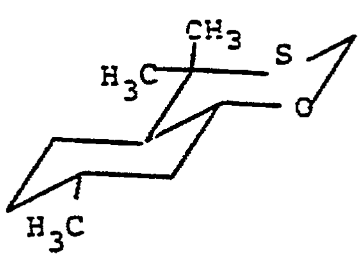

(III-1)

in 150 ml absolutem Tetrahydrofuran werden bei –78°C unter Stickstoffatmosphäre 45,5 ml (90 mMol) n-Butyl-Lithium in n-Hexan (1,98 molar) gegeben. Man erwärmt die Lösung kurzzeitig auf 0°C, kühlt dann erneut auf –78°C ab, tropft bei dieser Temperatur unter Rühren eine Lösung von 20,2 g (90 mMol) 2,2-Dimethyl-5-(4-Chlorphenyl)-pentan-3-on in 50 ml absolutem Tetrahydrofuran hinzu, erwärmt das Reaktionsgemisch dann langsam auf Raumtemperatur und rührt noch 30 Minuten bei 25°C nach. Zur Aufarbeitung gießt man das Reaktionsgemisch in 150 ml gesättigte, wäßrige Ammoniumchlorid-Lösung und extrahiert dreimal mit Methylenchlorid. Die vereinigten organischen Phasen werden über Natriumsulfat getrocknet und anschließend unter vermindertem Druck eingeengt. Man erhält auf diese Weise 37,6 g eines Produktes, das zu 98% aus den Diastereomeren der Formeln (IV-1) und (IV-2) besteht, wobei in dem Diastereomeren-Gemisch gemäß gaschromatographischer Analyse das R-Diastereomer der Formel (IV-1) zu 74% und das S-Diastereomer der Formel (IV-2) zu 26% enthalten ist.

$R_F$-Werte (Kieselgel; Petrolether: Diethylether = 5:1)
R-Diastereomer $R_F$ = 0,43
S-Diastereomer $R_F$ = 0,31

2. Stufe

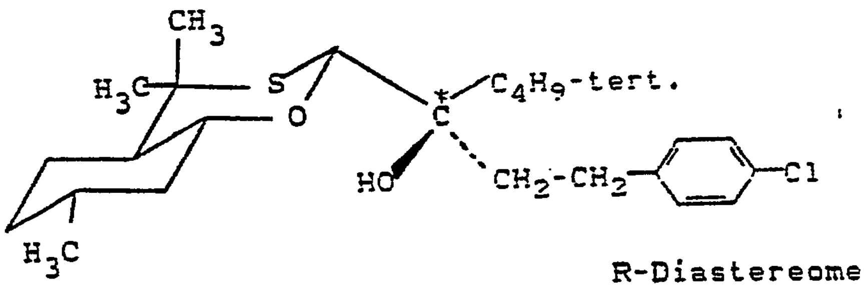

R-Diastereomer
(IV-1)

Jeweils 13 g des in der ersten Stufe erhaltenen Diastereomeren-Gemisches werden über eine Säule an Kieselgel chromatographiert, wobei als Laufmittel ein Gemisch aus Petrolether: Diethylether = 5:1 eingesetzt wird. Nach dem Einengen der eluierten Fraktionen erhält man

20,5 g (54% der Theorie) an R-Diastereomer der Formel (IV-1)
und
7,04 g (18% der Theorie) an S-Diastereomer der Formel (IV-2).

Die Substanzen liegen in Form von farblosen Ölen vor.

Gemäß gaschromatographischer Analyse ist das R-Diastereomer völlig diastereomeren-rein, während das S-Diastereomer noch 2–3% an R-Diastereomer enthält.

IR-Spektrum (NaCl):

Identisch für R- und S-Diastereomer; jeweils OH-Bande bei 3550 $cm^{-1}$.

Massenspektrum

Identisch für R- und S-Diastereomer m/e = 424 ($M^+$, fehlt); 367 (4%, $M^+$-tert.-Butyl); 199 (100%, Oxathian-Fragment).

$^1$H-NMR-Spektrum (360 MHz, $CDCl_3$)

R-Diastereomer:

δ = 0,92 (d, 3H, $\underline{CH_3}$–CH)
1,03 (s, 9H, tert.-Butyl)
1,29 und 1,44 (je s, je 3H, $\underline{CH_3}$–C–$\underline{CH_3}$)
2,59 und 3,09 (je d von t, J = 5,0; 13,1 Hz,

je 1H, $HO-\overset{|}{C}-\underline{CH_2}-$)

3,37 (s, 1H, $\underline{OH}$)

3,41 (d von t, 1H, –CH–O)

5,05 (s, 1H, S-CH-O)

7,17 –7,30 (m, 4H, arom. H)

S-Diastereomer:

δ = 0,93 (α, 3H, $CH_3$–CH)
1,02 (s, 9H, tert.-Butyl)
1,29 und 1,42 (je s, je 3H, $CH_3$–C–$CH_3$)
2,75 und 3,05 (je d von t, J = 4,9; 12,8 Hz,

je 1H, HO-C-$CH_2$-)

2,06 (s, 1H, OH)
3,36 (d von t, 1H, –CH-O–)

5,29 (s, 1H, -S-CH-O-)

7,15–7,28 (m, 4H, arom. H)

3. Stufe

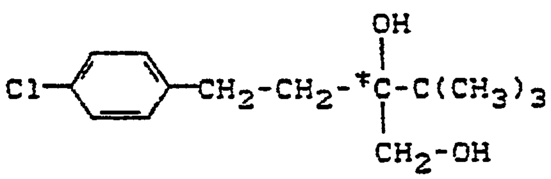

(VI-1)
R-Enantiomer

Eine Lösung von 17,9 g (42 mMol) des R-Diastereomeren der Formel (IV-1) in 75 ml Acetonitril wird unter Rühren in ein auf 40–50°C erwärmtes Gemisch aus 13,5 g (101 mMol) N-Chlor-succinimid, 14,3 g (84 mMol) Silbernitrat, 11,3 g (134 mMol) Natriumhydrogencarbonat, 250 ml Acetonitril und 50 ml Wasser gegeben. Man rührt noch 15 Minuten bei 40 bis 50°C, tropft dann nacheinander 38 ml gesättigte, wäßrige Natriumsulfit-Lösung und 38 ml gesättigte, wäßrige Natriumchlorid-Lösung hinzu, saugt ab und wäscht den Rückstand mit einem Gemisch aus Methylenchlorid/Hexan = 1:1.

Das Filtrat wird zweimal mit jeweils dem gleichen Volumen eines Gemisches aus Methylenchorid/Hexan = 1:1 extrahiert. Die organische Phase wird abgetrennt, mit gesättigter, wäßriger Natriumhydrogencarbonat-Lösung gewaschen über Natriumsulfat getrocknet, unter vermindertem Druck eingeengt und anschließend im Hochvakuum von noch enthaltenen Lösungsmittel-Resten befreit. Das dabei anfallende Produkt, das zu überwiegendem Anteil aus optisch aktivem α-Hydroxyaldehyd der Formel

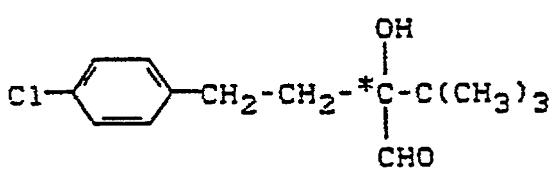

(V-1)
R-Enantiomer

besteht, wird in 75 ml Diethylether gelöst und bei Raumtemperatur unter Rühren in eine Suspension von 7,96 g (0,21 Mol) Lithium-aluminium-hydrid in 80 ml absolutem Diethylether getropft. Man erhitzt das Reaktionsgemisch 3 Stunden unter Rückfluß, tropft dann nacheinander 14 ml Essigester, 14 ml 4N wäßrige Natronlauge und 14 ml Wasser hinzu, saugt ab, wäscht mit Diethylether nach, trocknet die organische Phase über Natriumsulfat und engt unter vermindertem Druck ein.

Man erhält auf diese Weise 11,8 g eines Produktes, das gemäß gaschromatogrphischer Analyse zu 80% aus dem optisch aktiven Diol der Formel (VI-1) besteht.

IR-Spektrum (NaCl):
OH-Bande bei 3100–3600 $cm^{-1}$.

Massenspektrum

m/e = 256 ($M^+$, fehlt); 199 (18%, $M^+$ -tert.-Butyl), 181 (37%, $M^+$ -$H_2O$-tert.-Butyl); 125 (100%,

+ 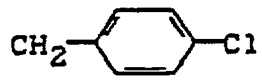 )

$^1$H-NMR-Spektrum (360 MHz, $CDCl_3$)

δ = 0,97 (s, 94, tert.-Butyl)
1,70–1,93 (m, 2H, $\underline{CH_2}$–Ph–Cl)

2,70-2,80 (m, 2H, HO-C-$\underline{CH_2}$-)

2,70–2,80 (m, 2H, HO–C–$\underline{CH_2}$–)
3,65 und 3,78 (dd, J = 11,0 Hz, 2H, –$\underline{CH_2}$OH)
7,13–7,35 (m, 4H, aromat. H)

Der bei der Reduktion mit Lithium-aluminium-hydrid anfallende Aluminiumhydroxid-Niederschlag wird in 500 ml 10%iger wäßriger Salzsäure gelöst. Die entstehende Lösung wird viermal mit Methylenchlorid extrahiert. Die vereinigten organischen Phasen werden über Natriumsulfat getrocknet und unter vermindertem Druck eingeengt. Man erhält auf diese Weise 5,02 g (64% der Therorie) an 1-Hydroxy-2-(1-Methyl-1-mercapto-ethyl)-5-methyl-cyclohexan in Form eines praktisch enantiomeren reinen Produktes.

4. Stufe

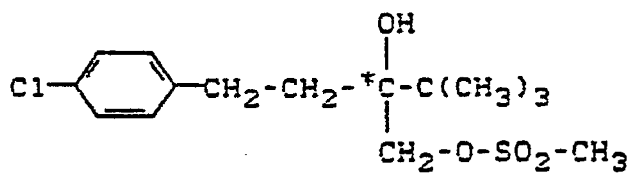

(VIII-1)
R-Enantiomer

In eine Lösung von 10,78 g (42 mMol) des R-Enantiomeren der Formel (VI-1) in 100 ml absolutem Methylenchlorid werden unter Stickstoffatmosphäre bei 0°C unter Rühren gleichzeitig eine Lösung von 4,80 g (42 mMol) Methansulfonsäurechlorid in 50 ml absolutem Methylenchlorid und eine Lösung von 4,2 g (42 mMol) Triethylamin in 50 ml absolutem Methylenchlorid eingetropft. Es wird 4 Stunden bei 0°C nachgerührt und dann auf 25°C erwärmt. Man extrahiert das Reaktionsgemisch nacheinander mit gesättigter, wäßriger Citronensäure-Lösung und mit gesättigter, wäßriger Natriumhydrogencarbonat-Lösung, trocknet über Natriumsulfat und engt unter vermindertem Druck ein. Man erhält auf diese Weise 14,0 g eines Produktes, das zu 80% aus dem R-Enantiomeren der Formel (VIII-1) besteht. Die Ausbeute errechnet sich danach zu 80% der Theorie.

Das Produkt wird ohne zusätzliche Reinigung weiter umgesetzt.

5. Stufe

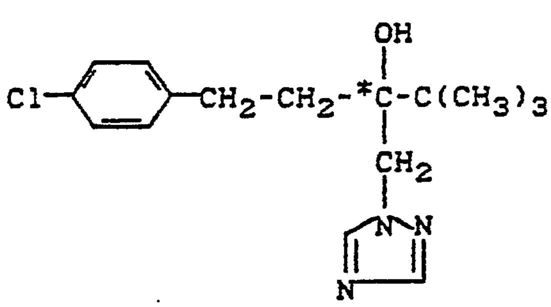

(I-1)
R-Enantiomer

Ein Gemisch aus 14,06 g (42 mMol) des R-Enantiomeren der Verbindung der Formel (VIII-1), 11,47 g (126 mMol) Triazol-Natriumsalz und 50 l Dimethylfomamid wird unter Stickstoffatmosphäre 6 Stunden bei 120°C gerührt. Danach wird unter vermindertem Druck eingeengt und der verbleibende Rückstand in ein Gemisch aus Wasser und Methylenchlorid gegeben. Man trennt die organische Phase ab, extrahiert die wäßrige Phase zweimal mit Methylenchlorid, trocknet die vereinigten organischen Phasen über Natriumsulfat und engt ein. Es verbleiben 11,40 g eines Produktes, das durch Säulenchromatographie an 350 g Kieselgel mit einem Gemisch aus Methylenchlorid/Methanol = 95:5 als Laufmittel gereinigt wird. Durch Einengen des Eluates erhält man 5,56 g eines Produktes, das gemäß gaschromatographischer Analyse zu 93–95% aus dem R-Enantiomeren des 2,2-Dimethyl-5-(4-chlorphenyl)-3-(1,2,4-triazol-1-yl-methyl)-pentan-3-ols besteht. Die Ausbeute errechnet sich daraus zu 43% der Theorie, bezogen auf das R-Diastereomer der Formel (IV-1).

$[\alpha]_D^{20} = + 34{,}1°$ (c = 1, $CHCl_3$):
Optische Reinheit ≥ 84%
Gehalt an S-Enantiomer ≦ 8%

Massenspektrum (ci)
m/e = 308 (100%, $M^+$ + H)

$^1$H-NMR-Spektrum (350 MHz, $CDCl_3$):

δ = 1,04 (s, 9H, tert.-Butyl)

1,70-2,00 (m, 2H, HO-C-$\underline{CH_2}$-)

1,70–2,00 (m, 2H, HO–C–$\underline{CH_2}$–)
3,37 (s, 1H, –$\underline{OH}$)
4,35 (s, 2H, –$\underline{CH_2}$-Triazol)
6,90–7,30 (m, 4H, aromat. H)
8,00 und 8,35 (je s, je 1H, Triazol-H)

Beispiel 2

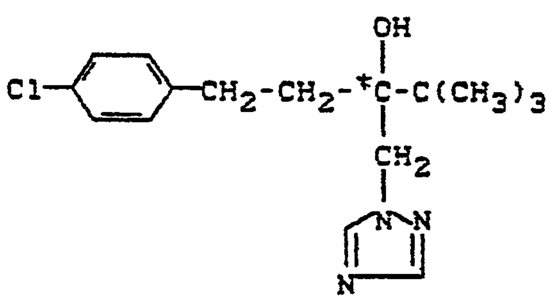

(I-2)
S-Enantiomer

Verfahrensvariante (b)

1. Stufe

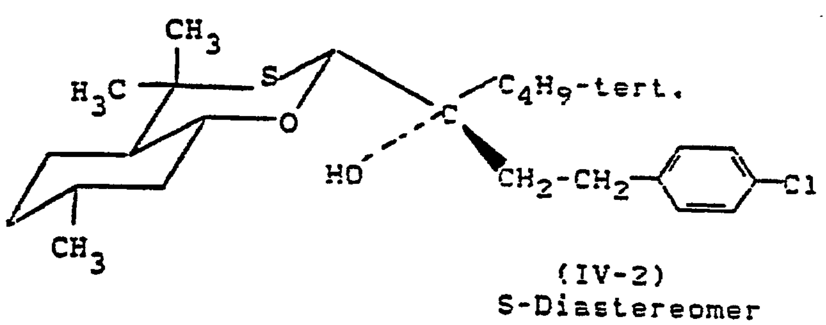

(IV-2)
S-Diastereomer

Zu einer Suspension von 5,30 g (0,22 Mol) Magnesium-Spänen in 10 ml absolutem Diethylether wird unter Stickstoffatmosphäre und unter Rühren eine Lösung von 48,3 g (0,22 Mol) 2-(4-Chlorphenyl)-ethylbromid in 100 ml absolutem Diethylether so zugetropft, daß das Reaktionsgemisch gleichmäßig siedet. Es wird 2 Stunden unter Rückfluß erhitzt, bis alles Magnesium aufgelöst ist. Man kühlt auf –78°C ab und tropft bei dieser Temperatur eine Lösung von 31,3 g (0,11 Mol) des Oxathian-Ketons der Formel

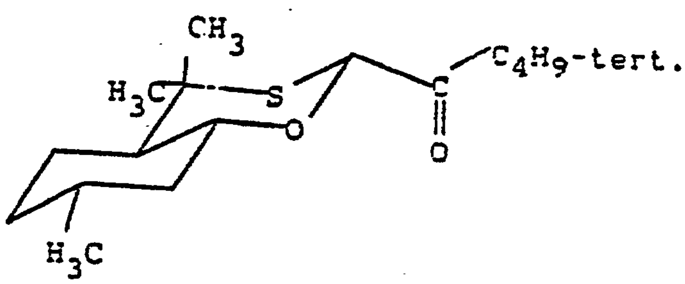

(X-1)

in 200 ml absolutem Diethylether hinzu.

Danach wird zunächst langsam auf Raumtemperatur erwärmt und dann 3 Stunden unter Rückfluß erhitzt. Zur Aufarbeitung gießt man das Reaktionsgemisch in 200 ml gesättigte, wäßrige Ammoniumchlorid-Lösung, trennt die organische Phase ab, extrahiert die wäßrige Phase dreimal mit Methylenchlorid, trocknet die vereinigten organischen Phasen über Natriumsulfat und engt unter vermindertem Druck ein. Als Rückstand verbleiben 116,8 g eines Produktes, das zu

47,6% aus dem S-Diastereomeren der Formel (IV-2),
1,13% aus dem R-Diastereomeren der Formel (IV-1),
30% aus dem 4-Chlor-ethylbenzol,
2% aus 2-(4-Chlorphenyl)-ethanol und zu
1% aus Di-(4-Chlorphenyl)-butan

besteht.

S-Diastereomer der Formel (IV-2) und R-Diastereomer der Formel (IV-1) sind im Verhältnis 97,7:2,3 (95,4% d.e.) vorhanden.

Durch Andestillieren im Hochvakuum bei 140°C werden niedrig siedende Anteile entfernt. Man erhält 40,2 g eines Produktes, das zu 80% aus dem S-Diastereomeren der Formel (IV-2) besteht. Das Produkt wird ohne zusätzliche Reinigung weiter umgesetzt.

IR-Spektrum (NaCl):
OH-Bande bei 3550 $cm^{-1}$

Massenspektrum:

m/e = 424 ($M^+$, fehlt); 367 (4%, $M^*$-tert.-Butyl);
199 (100%, Oxathian-Fragment).

$^1$H-NMR-Spektrum (360 MHz, $CDCl_3$):

δ = 0,93 (d, 3H, $CH_3$–CH)
1,02 (s, 9H, tert.-Butyl)
1,29 und 1,42 (je s, je 3H, $\underline{CH_3}$–C–$\underline{CH_3}$)
2,75 und 3,05 (je d von t, J = 4,9; 12,8 Hz,
je 1H, HO-C-$\underline{CH_2}$)
2,06 (s, 1H, $\underline{OH}$)
3,36 (d von +, 1H, –$\underline{CH}$–O–)
5,29 (s, 1H, -S-$\underline{CH}$-O-)
7,15–7,28 (m, 4H, arom. H)

2. Stufe

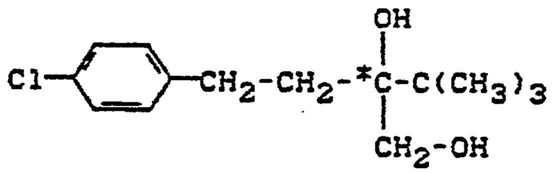

(VI-2)
S-Enantiomer

Eine Lösung von 40,2 g (0,11 Mol) des S-Diastereomeren der Formel (IV-2) in 150 ml absolutem Acetonitril wird unter Rühren in ein auf 40–50°C erwärmtes Gemisch aus 35,2 g (0,26 Mol) N-Chlor-succinimid, 37,3 g (0,22 mMol) Silbernitrat, 29,5 g (0,35 Mol) Natriumhydrogencarbonat, 500 ml Acetonitril und 100 ml Wasser gegeben. Man rührt noch 15 Minuten bei 40 bis 50°C, tropft dann nacheinander 82 ml gesättigte, wäßrige Natriumsulfit-Lösung und 82 ml gesättigte, wäßrige Natriumchlorid-Lösung hinzu, saugt ab und wäscht den Rückstand mit einem Gemisch aus Methylenchlorid/Hexan = 1:1.

Das Filtrat wird zweimal mit jeweils dem gleichen Volumen eines Gemisches aus Methylenchorid/Hexan = 1:1 extrahiert. die organische Phase wird abgetrennt, mit gesättigter, wäßriger Natriumhydrogencarbonat-Lösung gewaschen über Natriumsulfat getrocknet, unter vermindertem Druck eingeengt und anschließend im Hochvakuum von noch enthaltenen Lösungsmittel-Resten befreit. Das dabei anfallende Produkt, das zu überwiegendem Anteil aus optisch aktivem α-Hydroxyaldehyd der Formel

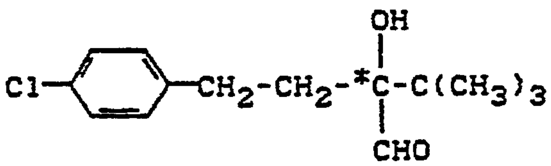

(V-2)
S-Enantiomer

besteht, wird in 100 ml absolutem Diethylether gelöst und bei Raumtemperatur unter Rühren in eine Suspension von 20,8 g (0,55 Mol) Lithium-aluminium-hydrid in 150 ml absolutem Diethylether getropft. Man erhitzt das Reaktionsgemisch 3 Stunden unter Rückfluß, tropft dann nacheinander 28 ml Essigester, 28 ml 4N wäßrige Natronlauge und 28 ml Wasser hinzu, saugt ab, wäscht mit Diethylether nach, trocknet die organische Phase über Natriumsulfat und engt unter vermindertem Druck ein.

Man erhält auf diese Weise 24,1 g eines Produktes, das gemäß gaschromatographischer Analyse zu 80% aus dem optisch aktiven Diol der Formel (VI-2) besteht.

IR-Spektrum (NaCl):
OH-Bande bei 3100–3600 $cm^{-1}$.

Massenspektrum

m/e = 256 ($M^+$, fehlt); 199 (18%, $M^+$ -tert.-Butyl), 181 (37%, $M^+$ -$H_2O$-tert.-Butyl); 125 (100%,

+ 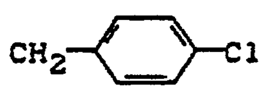 )

$^1$H-NMR-Spektrum (360 MHz, $CDCl_3$)

δ = 0,97 (s, 94, tert.-Butyl)
1,70–1,93 (m, 2H, –$\underline{CH_2}$–Ph–Cl)

2,70-2,80 (m, 2H, HO-C-$\underline{CH_2}$-)

3,65 und 3,78 (dd, J = 11,0 Hz, 2H, -$\underline{CH_2}$OH)
7,13–7,35 (m, 4H, aromat. H)

Der bei der Reduktion mit Lithium-aluminium-hydrid anfallende Aluminiumhydroxid-Niederschlag wird in 1 Liter 10%iger wäßriger Salzsäure gelöst. Die entstehende Lösung wird viermal mit Methylenchlorid extrahiert. Die vereinigten organischen Phasen werden über Natriumsulfat getrocknet und unter vermindertem Druck eingeengt. Man erhält auf diese Weise 12,2 g (59% der Theorie) an 1-Hydroxy-2-(1-methyl-1-mercapto-ethyl)-5-methyl-cyclohexan in Form eines enantiomerenreinen Produktes.

3. Stufe

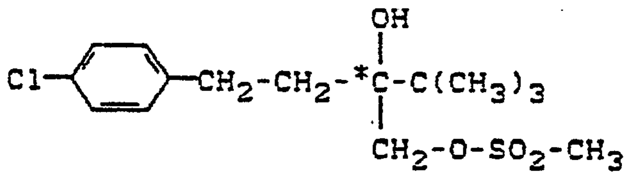

(VIII-2)
S-Enantiomer

In eine Lösung von 24,0 g (93,4 mMol) des S-Enantiomeren der Formel (VI-2) in 100 ml absolutem Methylenchlorid werden unter Stickstoffatmosphäre be 0°C unter Rühren gleichzeitig eine Lösung von 11,8 g (103 mMol) Methansulfonsäurechlorid in 50 ml absolutem Methylenchlorid und eine Lösung von 10,4 g (103 mMol) Triethylamin in 50 ml absolutem Methylenchlorid eingetropft. Es wird 4 Stunden bei 0°C nachgerührt und dann auf 25°C erwärmt. Man extrahiert das Reaktionsgemisch nacheinander mit gesättigter, wäßriger Citronensäure-Lösung und mit gesättigter wäßriger Natriumhydrogencarbonat-Lösung, trocknet über Natriumsulfat und engt unter vermindertem Druck ein. Man erhält auf diese Weise 27,97 g eines Produktes, das zu 80% aus dem S-Enantiomeren der Formel (VIII-2) besteht. Die Ausbeute errechnet sich danach zu 71% der Theorie.

Das Produkt wird ohne zusätzliche Reinigung weiter umgesetzt.

IR-Spektrum (NaCl):
OH-Bande bei 3540 $cm^{-1}$
$R{-}SO_2{-}OR$ : 1345 $cm^{-1}$ und 1175 $cm^{-1}$

4. Stufe

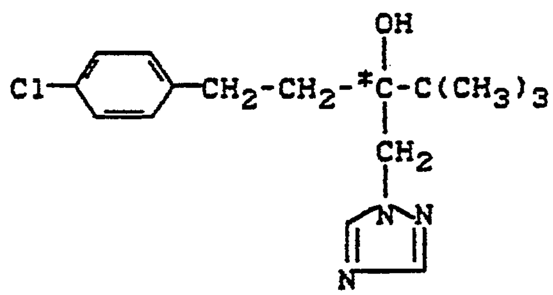

(I-2)
S-Enantiomer

Ein Gemisch aus 27,3 g (81,5 mMol) des S-Enantiomeren der Verbindung der Formel (VIII-2), 22,3 g (240 mMol) Triazol-Natriumsalz und 100 l Dimethylformamid wird unter Stickstoffatmosphäre 6 Stunden bei 120°C gerührt. Danach wird unter vermindertem Druck eingeengt und der verbleibende Rückstand in ein Gemisch aus Wasser und Methylenchlorid gegeben. Man trennt die organischen Phase ab, extrahiert die wäßrige Phase zweimal mit Methylenchlorid, trocknet die vereinigten organische Phasen über Natriumsulfat und engt ein. Es verbleiben 33,0 g eines Produktes, das durch Säulenchromatographie an 1000 g Kieselgel mit einem Gemisch aus Methylenchlorid/Methanol = 95:5 als Laufmittel gereinigt wird.

Durch Einengen des Eluates erhält man 12,4 g eines Produktes, das gemäß gaschromatographischer Analyse zu 95% aus dem S-Enantiomeren des 2,2-Dimethyl-5-(4-chlorphenyl)-3-(1,2,4-triazol-1-yl-methyl)-pentan-2-ols besteht. Die Ausbeute errechnet sich daraus zu 37% der Theorie, bezogen auf das S-Diastereomer der Formel (IV-2).

$[\alpha]_D^{20} = -34{,}0°$ (c = 1, $CHCl_3$)
Optische Reinheit ≥ 85% e.e.
Gehalt an S-Enantiomer ≦ 7%.

Massenspektrum: (ci)

m/e = 308 (100%, $M^+ + H$)

$^{1}$N-NMR-Spektrum (360 MHz, $CDCl_3$):

δ = 1,04 (s, 9H, tert.-Butyl)

1,70–2,00 (m, 2H, HO-C-$\underline{CH_2}$-)

2,30–2,60 (m, 2H-$\underline{CH_2}$-Ph-Cl)
3,37 (s, 1H, -$\underline{OH}$)
4,35 (s, 2H, -$\underline{CH_2}$-Triazol)
6,90–7,30 (m, 4H, aromat. H)
8,00 und 8,3 (je s, je 1H, Triazol-H)

Herstellung des Ausgangsproduktes der Formel

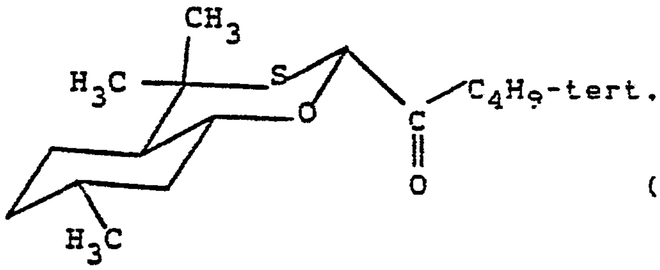

(X-1)

Zu einer Lösung von 40 g (0,20 mol) an Oxathian-Verbindung der Formel (III-1) in 150 ml absolutem Tetrahydrofuran werden bei –78°C unter Stickstoffatmosphäre 100 ml (0,20 mol) n-Butyllithium in n-Hexan (2,0 molar) gegeben. Man erwärmt die Lösung kurzzeitig auf 0°C, kühlt dann erneut auf –78°C ab, tropft bei dieser Temperatur eine Lösung von 22,2 g (0,26 mol) Pivalaldehyd in 50 ml absolutem Tetrahydrofuran hinzu, erwärmt das Reaktionsgemisch dann langsam auf Raumtemperatur und rührt noch 30 Minuten bei 25°C nach. Die Aufarbeitung erfolgt nach der im Beispiel 1, erste Stufe, angegebenen Weise. Man erhält 57,3 g (100% der Theorie) an Produkt der Formel

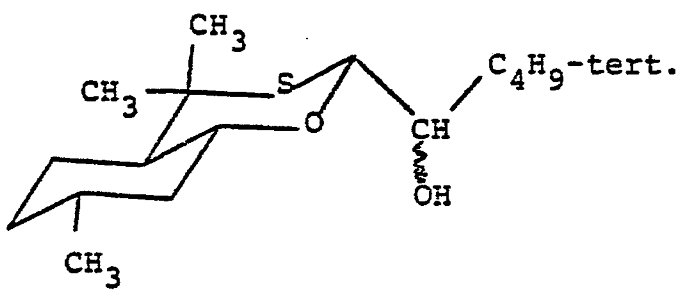

(XV-1)

in Form eines 60:40 Gemisches der Diastereomeren.

IR-Spektrum (NaCl).
OH-Bande bei 3600–3200 $cm^{-1}$

Zu einer Lösung von 14,8 g (0,19 mol) absolutem Dimethylsulfoxid in 100 ml absolutem Methylenchlorid wird bei –78°C unter Stickstoffatmosphäre und unter Rühren eine Lösung von 40,5 g (0,19 mol) Trifluoracetanhydrid in 50 ml absolutem Methylenchlorid hinzugetropft. Man rührt 15 Minuten bei –78°C nach, tropft dann eine Lösung von 51,3 g (0,18 mol) des Produktes der Formel (XV-1) in 50 ml absolutem Methylenchlorid hinzu, rührt 1 Stunde bei –78°C, tropft dann 61 ml (0,44 mol) Triethylamin bei –78°C hinzu und erwärmt langsam auf 0°C. Zur Aufarbeitung wird das Reaktionsgemisch in 500 ml 5%ige wäßrige Salzsäure gegossen. Man trennt die organische Phase ab, extrahiert die wäßrige Phase mit Methylenchlorid, wäscht die vereinigten organischen Phasen einmal mit wäßriger Kochsalz-Lösung, trocknet über Natriumsulfat und engt unter vermindertem Druck ein. Durch kurzzeitiges Erwärmen auf 100°C unter einem Druck von 0,1 mbar werden leicht flüchtige Bestandteile abdestilliert. Man erhält auf diese Weise 36,4 g (64% der Theorie) an der Verbindung der Formel (X-1).

IR-Sprektum (KBr):
CO-Bande bei 1700 $cm^{-1}$

$^{1}H$-NMR-Spektrum (360 MHz, $CDCl_3$):

δ =

0,92 (d, 3H, $\underline{CH_3}$-CH-)

1,24 (s, 9H, tert.-Butyl)
1,29 und 1,47 (je s, je 3H, $\underline{CH_3}$–C–$\underline{CH_3}$)
3,45 (d von t, 1H, $\underline{-CH}$–OR)

5,80 (s, 1H, -S-$\underline{CH}$-O-)

Herstellung weiterer Ausgangssubstanzen:

Beispiel 3

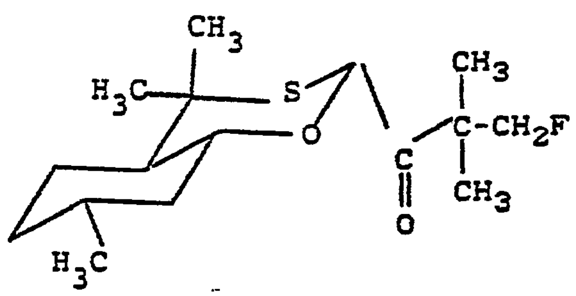

(X-2)

Zu einer Lösung von 16,32 g (81,5 mMol) des Oxathians der Formel (III-1) in 100 ml absolutem Tetrahydrofuran wird bei –78°C unter Stickstoffatmosphäre und unter Rühren eine Lösung von 39,7 g (81 mMol) n-Butyllithium (2,05 Mol) in n-Hexan zugegeben. Das Reaktionsgemisch wird kurzzeitig auf 0°C erwärmt und erneut auf –78°C abgekühlt.

Bei dieser Temperatur tropft man eine Lösung von 11,1 g (106 mMol) Monofluorpivaldehyd in 25 ml absolutem Tetrahydrofuran hinzu, erwärmt langsam auf 25°C und rührt 30 Minuten bei 25°C nach. Die Aufarbeitung erfolgt nach der im Beispiel 1, erste Stufe, angegebenen Weise. Man erhält 24,66 g (100% der Theorie) an Produkt der Formel

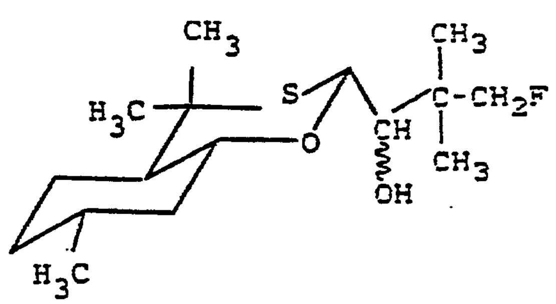

(XV-2)

in Form eines Diastereomeren-Gemisches, in dem das Verhältnis der Diastereomeren 56:44 beträgt.

IR-Spektrum (NaCl):
OH-Bande bei 3600–3200 $cm^{-1}$

Zu einer Lösung von 8,50 g (109 mMol) absolutem Dimethylsulfoxid in 50 ml absolutem Methylenchlorid wird bei –78°C unter Stickstoffatmosphäre und unter Rühren eine Lösung von 22,45 g (107 mMol) Trifluoracetanhydrid in 50 ml absolutem Methylenchlorid hinzugetropft. Man rührt 15 Minuten bei –78°C nach, tropft dann eine Lösung von 29,9 g (99 mMol) des Produktes der Formel (XV-2) in 50 ml absolutem Methylenchlorid hinzu, rührt 1 Stunde bei −78°C, tropft dann 34,3 ml (248 mMol) Triethylamin bei −78°C hinzu und erwärmt langsam auf 0°C. Zur Aufarbeitung wird das Reaktionsgemisch in 500 ml 5%ige wäßrige Salzsäure gegossen. Man trennt die organische Phase ab, extrahiert die wäßrige Phase mit Methylenchlorid, wäscht die vereinigten organischen Phasen einmal mit wäßriger Kochsalz-Lösung, trocknet über Natriumsulfat und engt unter vermindertem Druck ein. Durch kurzzeitiges Erhitzen auf 100°C unter einem Druck von 0,1 mbar werden leicht flüchtige Bestandteile abdestilliert. Man erhält auf diese Weise 22,92 g (77% der Theorie) an der Verbindung der Formel (X-2).

IR-Spektrum (NaCl):
CO-Bande bei 1720 $cm^{-1}$

$^1$N-NMR-Spektrum (360 MHz, $CDCl_3$)

δ = 0,93 (d, 3H, $CH_3$-CH-)
1,27, 1,30 und 1,47 (je s, je 3H, $CH_3$-)
3,48 (m, 1H, -CH-O-)
4,50 (m, 2H, -$CH_2$F)
5,78 (s,1H, -S-CH-O-)

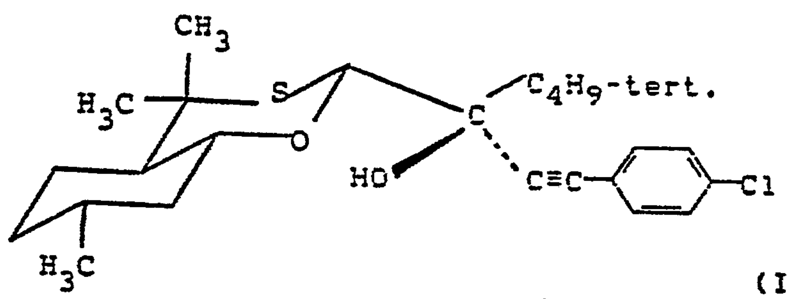

(IV-3)
S-Diastereomer

und

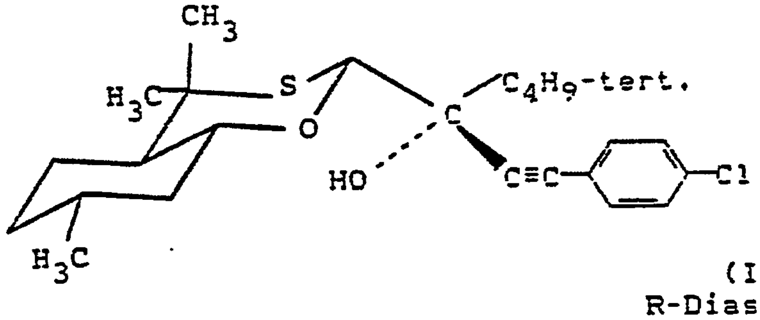

(IV-4)
R-Diastereomer

Zu einer Lösung von 3,41 g (25 mMol) 4-Chlorphenylacetylen in 20 ml absolutem Diethylether werden unter Stickstoffatmosphäre bei 0°C unter Rühren 9,75 ml (20 mMol) n-Butyl-lithium in n-Hexan (2,05 M) getropft. Nach 10 Minuten fügt man 5,16 g (20 mMol) Magnesiumbromid-Etherat hinzu und rührt 15 Minuten bei 0°C nach. Nach dem Abkühlen des Reaktionsgemisches auf −78°C wird bei dieser Temperatur eine Lösung von 3,02 g (10 mMol) des Oxathian-ketons der Formel (X-2) in 10 ml absolutem Ether zugetropft.

Das Reaktionsgemisch wird langsam auf Raumtemperatur erwärmt und noch 4 Stunden unter Rückfluß erwärmt. Zur Aufarbeitung gießt man das Reaktionsgemisch in 100 ml wäßrige Ammoniumchlorid-Lösung,

trennt die organische Phase ab, extrahiert die wäßrige Phase dreimal mit Methylenchlorid, trocknet über Natriumsulfat und engt unter vermindertem Druck ein. Man erhält in quantitativer Ausbeute ein Gemisch aus den beiden Diastereomeren der Formeln (IV-3) und (IV-4), in dem das Verhältnis R:S-Diastereomer = 95:4,2 (91,6% d.e.) beträgt.

IR-Spektrum (NaCl):
OH-Bande bei 3600–3200 $cm^{-1}$

Massenspektrum (Ci):
m/e = 421 (100%, $MH^+$-$H_2O$)

<u>Beispiel 4</u>

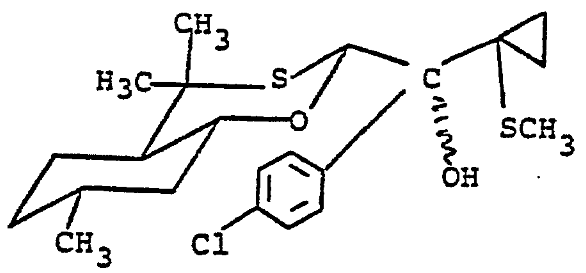

(IV-5)

Zu einer Lösung von 12,00 g (60 mMol) des Oxathians der Formel (III-1) in 70 ml absolutem Tetrahydrofuran werden bei –78°C unter Stickstoffatmosphäre und unter Rühren 29,3 ml n-Butyl-lithium in n-Hexan (2,05 M) gegeben. Das Reaktionsgemisch wird kurzzeitig auf 0°C erwärmt und erneut auf –78°C abgekühlt.

Bei dieser Temperatur tropft man eine Lösung von 13,60 (60 mMol) (4-Chlorphenyl)-(1-methylmercapto-cycloprop-1-yl)-keton in 40 ml absolutem Tetrahydrofuran hinzu, erwärmt langsam auf Raumtemperatur und rührt 30 Minuten bei Raumtemperatur nach. Die Aufarbeitung erfolgt nach der im Beispiel 1, erste Stufe, angegebenen Weise. Man erhält 25,2 g (98% der Theorie) an Diastereomeren-Gemisch, in dem die beiden Diastereomeren im Verhältnis 61:39 vorliegen.

IR-Spektrum (NaCl):
OH-Bande 3600–3200 $cm^{-1}$
= CH 3090 und 3030 $cm^{-1}$
C=C 1600 und 1590 $cm^{-1}$

Die Diastereomeren werden durch Säulenchromatographie an Kieselgel (Laufmittel: Chlorbenzol) getrennt.

<u>Diastereomer I</u>

1H-NMR-Spektrum (360 MHz, $CDCl_3$)

δ = 2,96 (s, 1H, O-<u>H</u>)
3,59 (m, 1H, -<u>CH</u>-OR)
6,10 (s, 1H, -S-<u>CH</u>-O-)
7,25-7,63 (m, 4H, aromat. H)

Diastereomer II

δ = 2,87 (s, 1H, -O-H)

3,53 (m, 1H, -CH-OR)

6,20 (s, 1H, -S-CH-O-)

7,25-7,63 (m, 4H, aromat. H)

**Patentansprüche**

1. Verfahren zur Herstellung von optisch aktiven 2-Hydroxyethyl-azol-Derivaten der Formel

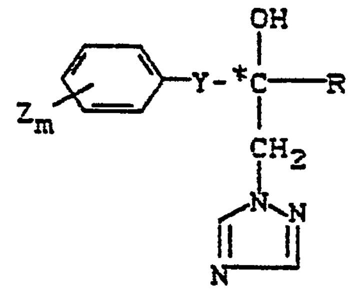

(I)

in welcher
R für gegebenenfalls durch Halogen, Alkoxy mit 1 bis 4 Kohlenstoffatomen, Alkylthio mit 1 bis 4 Kohlenstoffatomen, die CHO-Gruppe und deren Derivate, Phenoxy und/oder Phenyl substituiertes geradkettiges oder verzweigtes Alkyl mit 1 bis 6 Kohlenstoffatomen, für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen und/oder Halogen substituiertes Cycloalkyl mit 3 bis 8 Kohlenstoffatomen oder für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, und/oder Halogen substituiertes Phenyl steht,
Y für die Gruppierungen $-CH_2-CH_2-$, $-CH=CH-$ oder $-C\equiv C-$ steht oder für eine direkte Bindung steht,
Z für Fluor, Chlor, Brom, Alkyl mit 1 bis 4 Kohlenstoffatomen, Cycloalkyl mit 3 bis 8 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und
m für die Zahlen 0, 1, 2 oder 3 steht, dadurch gekennzeichnet, daß man

a) in einer ersten Stufe Ketone der Formel

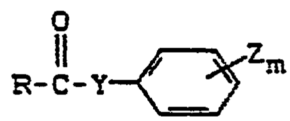

(II)

in welcher
R, Y, Z und m die oben angegebene Bedeutung haben, mit enantiomeren-reinen Oxathianen der Formel

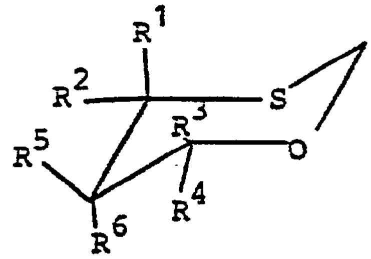

(III)

in welcher

$R^1$, $R^2$, $R^3$, $R^4$, $R^5$ und $R^6$ für Wasserstoff oder Alkyl mit 1 bis 4 Kohlenstoffatomen stehen, wobei jedoch mindestens einer dieser Reste für Alkyl mit 1 bis 4 Kohlenstoffatomen steht, oder

$R^4$ und $R^5$ auch gemeinsam für gegebenenfalls ein- bis dreifach, gleichartig oder verschieden durch Alkyl mit 1 bis 4 Kohlenstoffatomen substituiertes Alkandiyl mit 3 bis 6 Kohlenstoffatomen stehen oder auch

$R^4$ und $R^5$ gemeinsam mit den angrenzenden Kohlenstoffatomen für einen gegebenenfalls ein- bis dreifach, gleichartig oder verschieden durch Alkyl mit 1 bis 4 Kohlenstoffatomen substituierten, anellierten bicyclischen Kohlenwasserstoff mit 7 oder 8 Kohlenstoffatomen stehen,

in Gegenwart einer stark basischen metallorganischen Verbindung sowie in Gegenwart eines Verdünnungsmittels bei Temperaturen zwischen –80°C und +120°C umsetzt, dann in einer <u>zweiten Stufe</u> das so erhaltene Gemisch von diastereomeren Verbindungen der Formeln

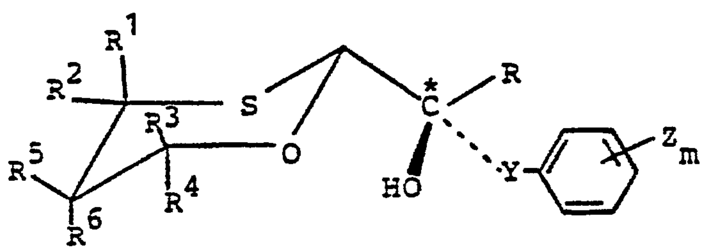

(IV-a)

und

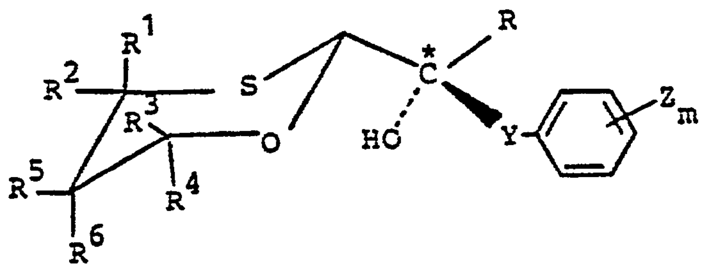

in welcher

R, $R^1$, $R^2$, $R^3$, $R^4$, $R^5$, $R^6$, Y, Z und m die oben angegebene Bedeutung haben, aufgrund ihrer unterschiedlichen physikalischen Eigenschaften trennt, danach in einer <u>dritten Stufe</u> das jeweils gewünschte Diastereomere der Formel (IV-a) oder (IV-b) zunächst mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischsn 0°C und 100°C umsetzt und die dabei entstehenden optisch aktiven α-Hydroxyaldehyde der Formel

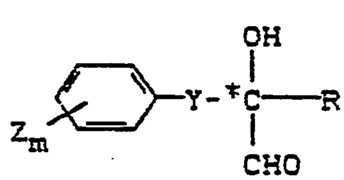

(V)

in welcher

R, Z, Y und m die oben angegebene Bedeutung haben, mit einem zur Reduktion von Aldehyden geeigneten Reagenz in Gegenwart eines Verdünnungsmittels bei Temperaturen zwischen –20°C und

+100°C umsetzt, weiterhin in einer vierten Stufe die so erhaltenen optisch aktiven Diole der Formel

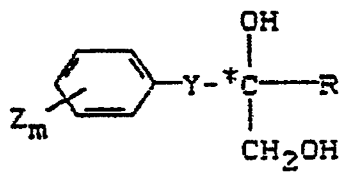

(VI)

in welcher
R, Y, Z und m die oben angegebene Bedeutung haben, mit Sulfonsäure-Derivaten der Formel,

$$R^7\text{–}SO_2\text{–Hal} \quad (VII)$$

in welcher
$R^7$ für Alkyl mit 1 bis 4 Kohlenstoffatomen, Halogenalkyl mit 1 oder 2 Kohlenstoffatomen und 1 bis 5 Halogenatomen oder für gegebenenfalls durch Methyl substituiertes Phenyl steht und
Hal für Chlor oder Brom steht, in Gegenwart eines Verdünnungsmittels und in Gegenwart eines Säurebindemittels bei Temperaturen zwischen 0°C und 100°C umsetzt und schließlich in einer fünften Stufe die entstandenen optisch aktiven Verbindungen der Formel

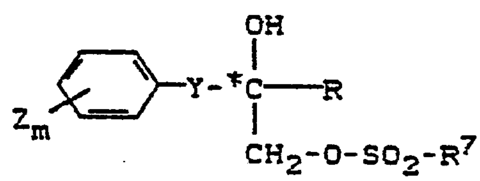

(VIII)

in welcher
R, $R^7$, Y, Z und m die oben angegebene Bedeutung haben, mit Triazol-Salzen der Formel

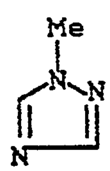

(IX)

in welcher
Me für Natrium oder Kalium steht, in Gegenwart eines Verdünnungsmittels und gegebenenfalls in Gegenwart eines Säurebindemittels bei Temperaturen zwischen 20°C und 150°C umsetzt, oder
b) in einer ersten Stufe enantiomerenreine Oxathian-Ketone der Formel

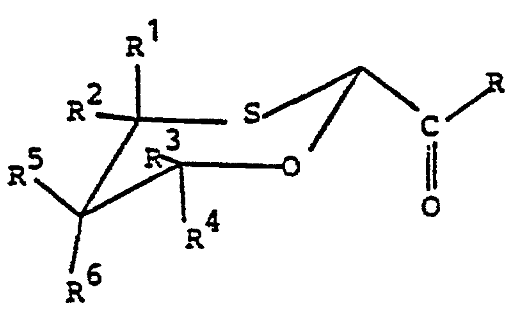

(X)

in welcher
R, $R^1$, $R^2$, $R^3$, $R^4$, $R^5$ und $R^6$ die oben angegebene Bedeutung haben mit metallorganischen Verbindungen der Formel

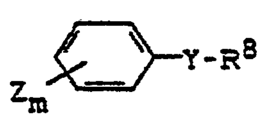

(XI)

in welcher

Y, Z und m die oben angegebene Bedeutung haben und
$R^8$ für Lithium, Natrium, Kalium, Titan-isopropylat, oder einen Rest der Formel $Me^1X$ steht, worin $Me^1$ für Magnesium oder Zink steht und X für Chlor, Brom oder Iod steht, in Gegenwart eines Verdünnungsmittels bei Temperaturen zwischen –78°C und +100°C umsetzt, danach in einer zweiten Stufe das bevorzugt entstandene Diastereomere der Formel

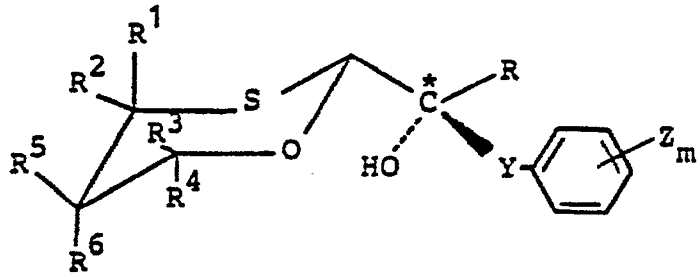

(IV-b)

in welcher
R, $R^1$, $R^2$, $R^3$, $R^4$, $R^5$, $R^6$, Y, Z und m die oben angegebene Bedeutung haben, gegebenenfalls nach vorheriger chromatographischer Abtrennung des in untergeordnetem Maße entstandenen Diastereomeren der Formel (IV-a) zunächst mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0°C und 100°C umsetzt und dann in gleicher Weise wie im Falle der Verfahrensvariante (a) weiterarbeitet, oder
c) in einer ersten Stufe enantiomeren-reine Oxathian-Ketone der Formel

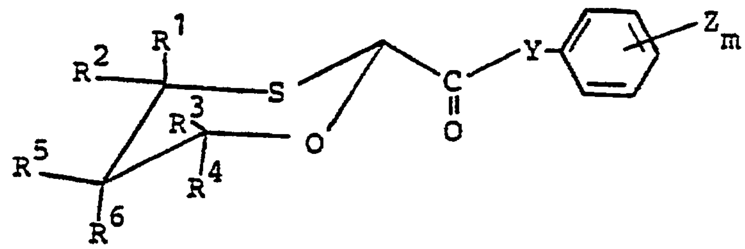

(XII)

in welcher
$R^1$, $R^2$, $R^3$, $R^4$, $R^5$, $R^6$, Y, Z und m die oben angegebene Bedeutung haben, mit metallorganischen Verbindungen der Formel

$$R-R^8 \quad (XIII)$$

in welcher
R und $R^8$ die oben angegebene Bedeutung haben, in Gegenwart eines Verdünnungsmittels bei Temperaturen zwischen –78°C und +100°C umsetzt,
danach in einer zweiten Stufe das bevorzugt entstandene Diastereomere der Formel

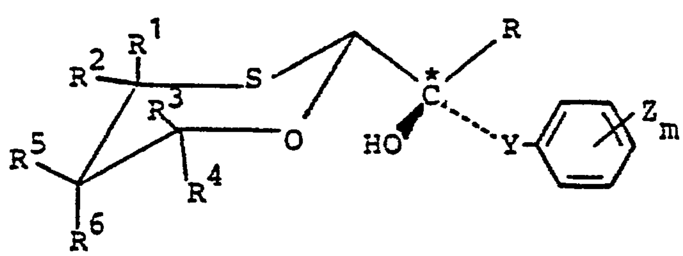

(IV-a)

in welcher
R, $R^1$, $R^2$, $R^3$, $R^4$, $R^5$, $R^6$, Y, Z und m die oben angegebene Bedeutung haben, gegebenenfalls nach vorheriger chromatographischer Abtrennung des in untergeordnetem Maße entstandenen Diastereomeren der Formel (IV-b) zunächst mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0°C und 100°C umsetzt und dann in gleicher Weise wie im Falle der Verfahrensvariante (a) weiterarbeitet.

2. Verfahren gemäß Anspruch 1, dadurch gekennzeichnet, daß man als Ausgangsstoff der Formel (II) das Keton der Formel

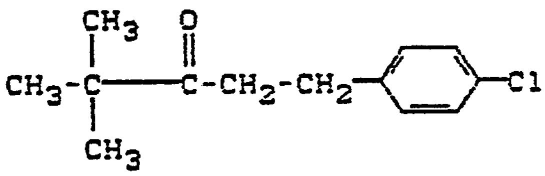

einsetzt.

3. Verfahren gemäß Anspruch 1, dadurch gekennzeichnet, daß man als Ausgangsstoff der Formel (III) das Oxathian der Formel

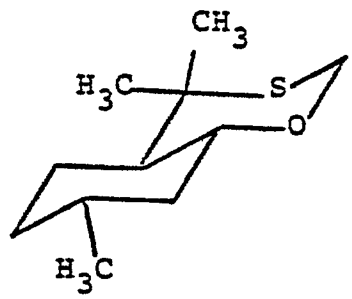

einsetzt.

4. Verfahren gemäß Anspruch 1, dadurch gekennzeichnet, daß man als Ausgangsstoff der Formel (X) das Oxathian-Keton der Formel

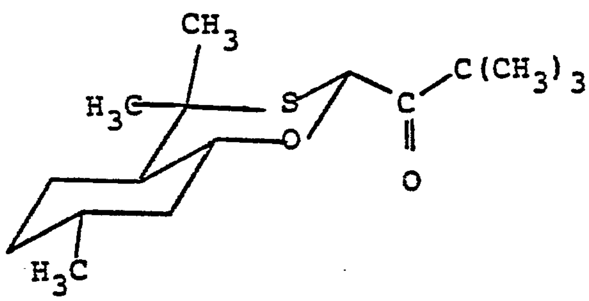

einsetzt.

5. Verfahren gemäß Anspruch 1, dadurch gekennzeichnet, daß man als Ausgangsstoff der Formel (XII) das Oxathian-Keton der Formel

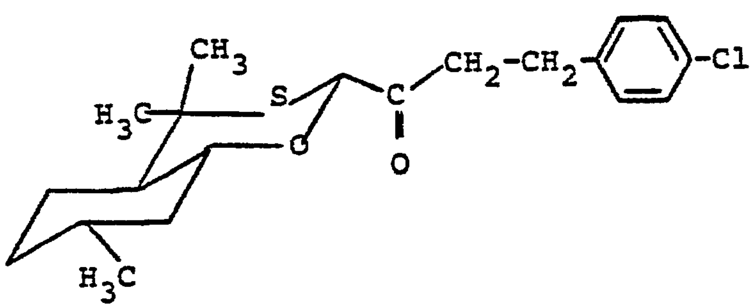

einsetzt.

## Claims

1. Process for the preparation of optically active 2-hydroxyethyl-azole derivatives of the formula

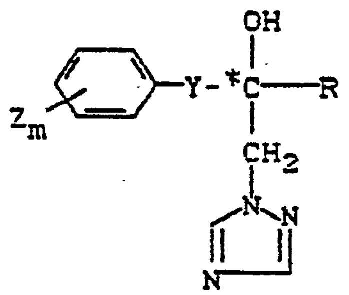

(I)

in which
R represents straight-chain or branched alkyl, having 1 to 6 carbon atoms, which is optionally substituted by halogen, alkoxy having 1 to 4 carbon atoms, alkylthio having 1 to 4 carbon atoms, the CHO group and its derivatives, phenoxy and/or phenyl, or cycloalkyl, having 3 to 8 carbon atoms, which is optionally substituted by alkyl having 1 to 4 carbon atoms, alkoxy having 1 to 4 carbon atoms, alkylthio having 1 to 4 carbon atoms and/or halogen, or phenyl which is optionally substituted by alkyl having 1 to 4 carbon atoms, alkoxy having 1 to 4 carbon atoms, alkylthio having 1 to 4 carbon atoms and/or halogen,
Y represents the $-CH_2-CH_2-$, $-CH=CH-$ or $-C{\equiv}C-$ groups or represents a direct bond,
Z represents fluorine, chlorine, bromine, alkyl having 1 to 4 carbon atoms, cycloalkyl having 3 to 8 carbon atoms, alkoxy having 1 to 4 carbon atoms, alkylthio having 1 to 4 carbon atoms, halogenalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl which is optionally substituted by halogen and/or alkyl having 1 to 4 carbon atoms, phenoxy which is optionally substituted by halogen and/or alkyl having 1 to 4 carbon atoms, phenylalkyl, having 1 or 2 carbon atoms in the alkyl part, which is optionally substituted by halogen and/or alkyl having 1 to 4 carbon atoms, or phenylalkoxy, having 1 or 2 carbon atoms in the alkoxy part, which is optionally substituted by halogen and/or alkyl having 1 to 4 carbon atoms, and
m represents the numbers 0, 1, 2 or 3, characterized in that

a) in a <u>first stage</u>, ketones of the formula

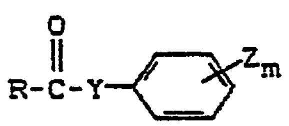

(II)

in which
R, Y, Z and m have the abovementioned meaning, are reacted with enantiomerically pure oxathianes of the formula

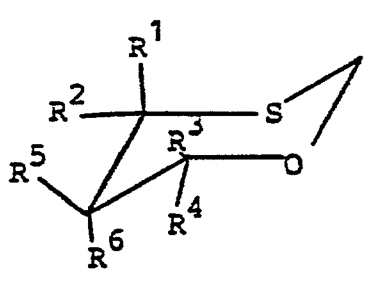

(III)

in which
$R^1$, $R^2$, $R^3$, $R^4$, $R^5$ and $R^6$ represent hydrogen or alkyl having 1–4 carbon atoms, but at least one of the radicals represents alkyl having 1–4 carbon atoms, or else
$R^4$ and $R^5$, together, represent alkanediyl, having 3 to 6 carbon atoms, which is optionally monosubstituted to trisubstituted by alkyl having 1 to 4 carbon atoms, or else
$R^4$ and $R^5$, together with the neighbouring carbon atoms, represent a bicyclic hydrocarbon radical, having 7 or 8 carbon atoms, which is optionally monosubstituted to trisubstituted by identical or different alkyl substituents having 1 to 4 carbon atoms, in the presence of a strongly basic organometallic

compound, and in the presence of a diluent, at temperatures between –80°C and +120°C, then, in a <u>second stage</u>, the mixture of diastereomeric compounds of the formulae

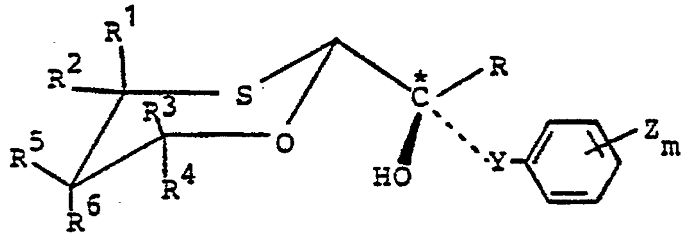

(IV-a)

and

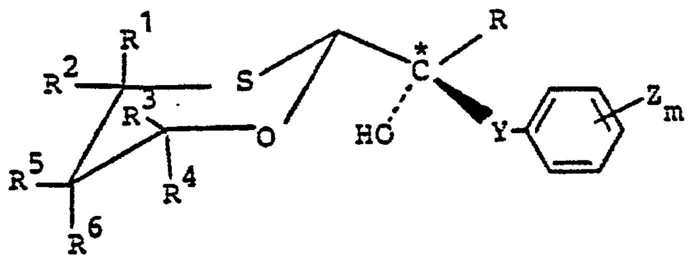

in which

R, $R^1$, $R^2$, $R^3$, $R^4$, $R^5$, $R^6$, Y, Z and m have the abovementioned meaning, thus obtained is separated as a result of their different physical properties, then, in a <u>third stage</u>, the diastereomer of the formula (IV-a) or (IV-b) desired in each case is initially reacted with a reagent which is suitable for the cleavage of oxathiane compounds, in the presence of a diluent and if appropriate in the presence of an acid-binding agent at temperatures between 0°C and 100°C, and the optically active α-hydroxyaldehydes of the formula

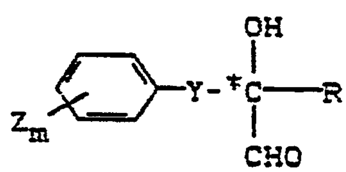

(V)

in which

R, Z, Y and m have the abovementioned meaning, produced during this are reacted with a reagent which is suitable for the reduction of aldehydes, in the presence of a diluent at temperatures between –20°C and +100°C, furthermore, in a <u>fourth stage</u>, the optically active diols of the formula

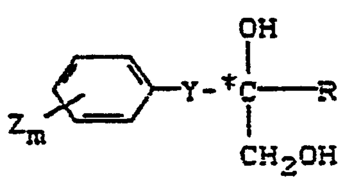

(VI)

in which

R, Y, Z and m have the abovementioned meaning, thus obtained are reacted with sulphonic acid derivatives of the formula

$$R^7\text{–}SO_2\text{–}Hal \quad (VII)$$

in which

$R^7$ represents alkyl having 1 or 2 carbon atoms and 1 to 5 halogen atoms, halogenoalkyl or optionally having 1 to 4 carbon atoms substituted phenyl, and

Hal represents methyl-chlorine or bromine, in the presence of a diluent and in the presence of an acid-binding agent at temperatures between 0°C and 100°C, and finally, in a <u>fifth stage</u>, the resultant optically active compounds of the formula

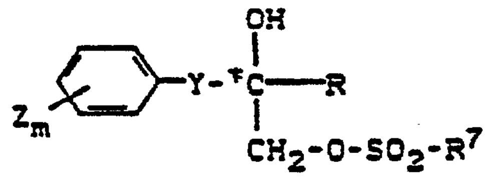

(VIII)

in which R, $R^7$, Y, Z and m have the abovementioned meaning, are reacted with triazole salts of the formula

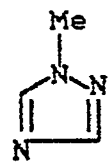

(IX)

in which Me represents sodium or potassium, in the presence of a diluent and if appropriate in the presence of an acid-binding agent at temperatures between 20°C and 150°C, or

b) in a <u>first stage</u>, enantiomerically pure oxathiane ketones of the formula

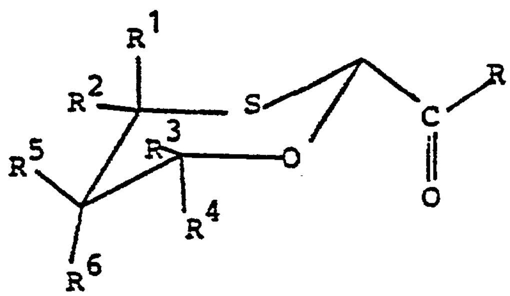

(X)

in which R, $R^1$, $R^2$, $R^3$, $R^4$, $R^5$ and $R^6$ have the abovementioned meaning, are reacted with organometallic compounds of the formula

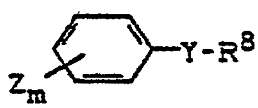

(XI)

in which Y, Z and m have the abovementioned meaning, and $R^8$ represents lithium, sodium, potassium or a radical of the formula $Me^1X$, in which $Me^1$ represents magnesium or zinc and X represents chlorine, bromine or iodine, in the presence of a diluent at temperatures between –78°C and +100°C, then, in a <u>second stage</u>, the diastereomer of the formula

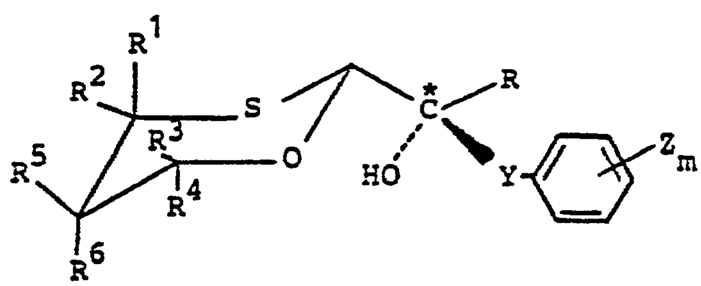

(IV-b)

in which R, $R^1$, $R^2$, $R^3$, $R^4$, $R^5$, $R^6$, Y, Z and m have the abovementioned meaning, produced preferentially is initially reacted, if appropriate after prior chromatographic separation of the diastereomer of the formula (IV-a) produced to a minor extent, with a reagent which is suitable for the cleavage of oxathiane

compounds, in the presence of a diluent and if appropriate in the presence of an acid-binding agent at temperatures between 0°C and 100°C, and then the further procedure is carried out in the same fashion as in the case of process version (a), or

c) in a first stage, enantiomerically pure oxathiane ketones of the formula

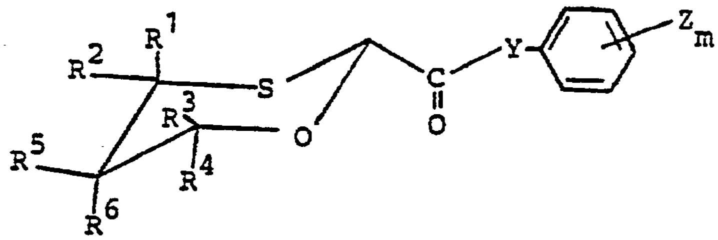

(XII)

in which

$R^1$, $R^2$, $R^3$, $R^4$, $R^5$, $R^6$, Y, Z and m have the abovementioned meaning, are reacted with organometallic compounds of the formula

$$R\text{–}R^8 \quad (XIII)$$

in which

R and $R^8$ have the abovementioned meaning, in the presence of a diluent at temperatures between −78°C and +100°C, then, in a second stage, the diastereomer of the formula

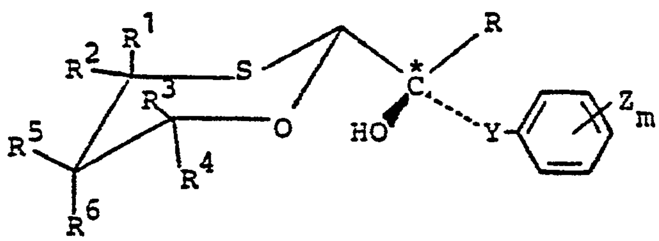

(IV-a)

in which

R, $R^1$, $R^2$, $R^3$, $R^4$, $R^5$, $R^6$, Y, Z and m have the abovementioned meaning, produced preferentially is initially reacted, if appropriate after prior chromatographic separation of the diastereomer of the formula (IV-b) produced to a minor extent, with a reagent which is suitable for the cleavage of oxathiane compounds, in the presence of a diluent and if appropriate in the presence of an acid-binding agent at temperatures between 0°C and 100°C, and then the further procedure is carried out in the same fashion as in the case of process version (a).

2. Process according, to claim 1, characterized in that the ketone of the formula

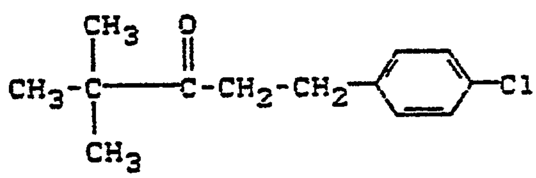

is employed as starting material of the formula (II).

3. Process according to claim 1, characterized in that the oxathiane of the formula (III)

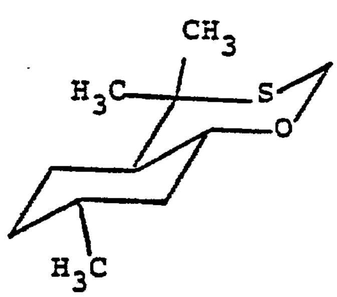

4. Process according to Claim 1, characterized in that the oxathiane ketone of the formula

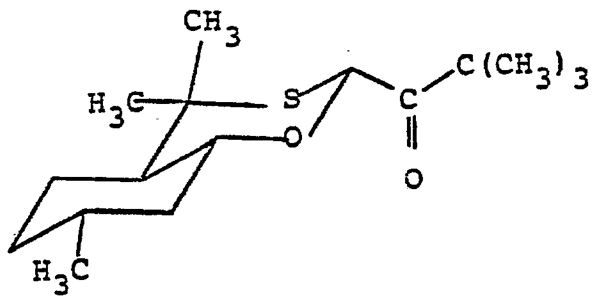

is employed as starting material of the formula (X).

5. Process according to Claim 1, characterized in that the oxathiane ketone of the formula

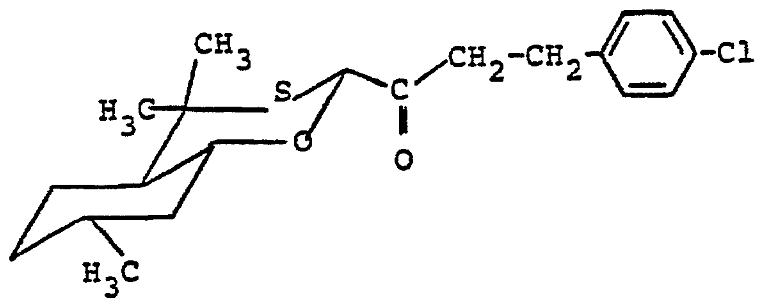

is employed as starting material of the formula (XII).

**Revendications**

1. Procédé de préparation de dérivés de 2-hydroxyéthyl-azole optiquement actifs de formule

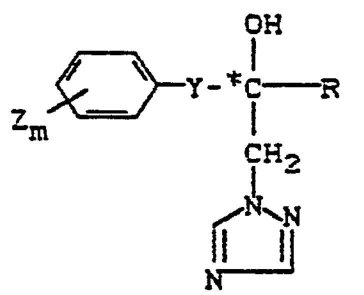

(I)

dans laquelle
R représente un groupe alkyle à chaîne droite ou ramifiée, contenant 1 à 6 atomes de carbone, éventuellement substitué par un atome d'halogène, par un groupe alcoxy contenant 1 à 4 atomes de carbone, par un groupe alkylthio contenant 1 à 4 atomes de carbone, par le groupe –CHO et ses dérivés, par un groupe phénoxy et/ou par un groupe phényle, un groupe cycloalkyle contenant 3 à 8 atomes de carbone, éventuellement substitué par un groupe alkyle contenant 1 à 4 atomes de carbone, par un groupe alcoxy contenant 1 à 4 atomes de carbone, par un groupe alkylthio contenant 1 à 4 atomes de carbone et/ou par un atome d'halogène, ou un groupe phényle éventuellement substitué par un groupe alkyle contenant 1 à 4 atomes de carbone, par un groupe alkoxy contenant 1 à 4 atomes de carbone, par un groupe alkylthio contenant 1 à 4 atomes de carbone et/ou par un atome d'halogène,
Y représente les groupements $-CH_2-CH_2-$, $-CH=CH-$ ou $-C\equiv C-$ ou encore une liaison directe,
Z représente un atome de fluor, un atome de chlore, un atome de brome, un groupe alkyle contenant 1 à 4 atomes de carbone, un groupe cycloalkyle contenant 3 à 8 atomes de carbone, un groupe alcoxy contenant 1 à 4 atomes de carbone, un groupe alkylthio contenant 1 à 4 atomes de carbone, un groupe halogénalkyle contenant 1 ou 2 atomes de carbone et 1 à 5 atomes d'halogènes identiques ou différents, un groupe halogénalcoxy contenant 1 ou 2 atomes de carbone et 1 à 5 atomes d'halogènes identiques ou différents, un groupe halogénalkylthio contenant 1 ou 2 atomes de carbone et 1 à 5 atomes d'halogènes

identiques ou différents, un groupe phényle éventuellement substitué par un atome d'halogène et/ou par un groupe alkyle contenant 1 à 4 atomes de carbone, un groupe phénoxy éventuellement substitué par un atome d'halogène et/ou par un groupe alkyle contenant 1 à 4 atomes de carbone, un groupe phénylalkyle contenant 1 ou 2 atomes de carbone dans la fraction alkyle, éventuellement substitué par un atome d'halogène et/ou par un groupe alkyle contenant 1 à 4 atomes de carbone, ou un groupe phénylalcoxy contenant 1 ou 2 atomes de carbone dans la fraction alcoxy, éventuellement substitué par un atome d'halogène et/ou par un groupe alkyle contenant 1 à 4 atomes de carbone, tandis que
m est égal à 0, 1, 2 ou 3, caractérisé en ce que l'on fait réagir

a) dans une première étape des cétones de formule

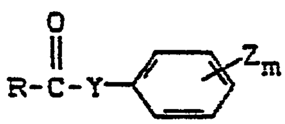

(II)

dans laquelle
R, Y, Z et m ont les significations indiquées ci-dessus, avec des oxathiannes énantiomériquement purs de formule

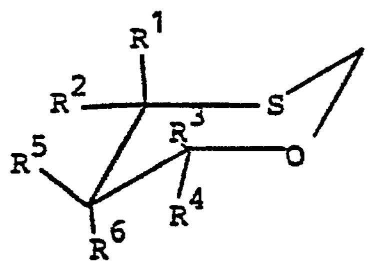

(III)

dans laquelle
$R^1$, $R^2$, $R^3$, $R^4$, $R^5$ et $R^6$ représentent chacun un atome d'hydrogène ou un groupe alkyle contenant 1 à 4 atomes de carbone, un de ces radicaux au moins représentant toutefois un groupe alkyle contenant 1 à 4 atomes de carbone,
ou
$R^4$ et $R^5$ représentent également ensemble un groupe alcanediyle contenant 3 à 6 atomes de carbone, éventuellement substitué une à trois fois, de manière identique ou différente, par un groupe alkyle contenant 1 à 4 atomes de carbone, ou encore
$R^4$ et $R^5$ représentent ensemble avec les atomes de carbone accolés, un hydrocarbure bicyclique condensé contenant 7 ou 8 atomes de carbone, éventuellement substitué une à trois fois, de manière identique ou différente, par un groupe alkyle contenant 1 à 4 atomes de carbone, en présence d'un composé organométallique fortement basique, ainsi qu'en présence d'un diluant, à des températures comprises entre −80°C et +120°C, puis, dans une deuxième étape, on sépare le mélange ainsi obtenu constitué de composés diastéréoisomères de formules

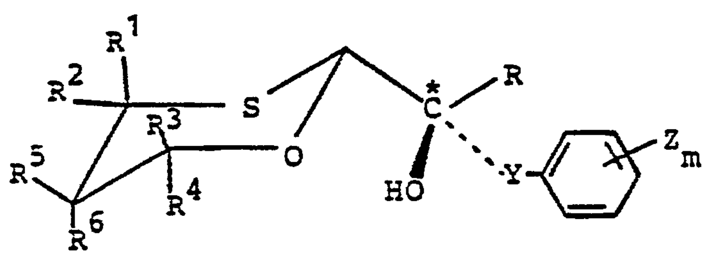

(IV-a)

et

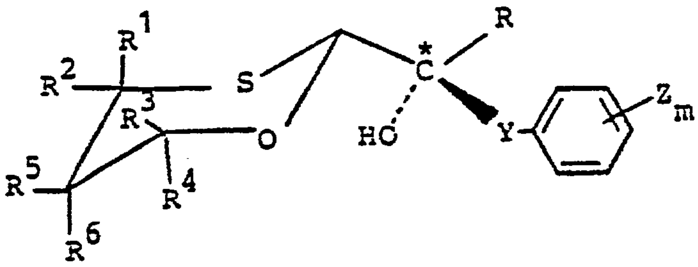

dans lesquelles
R, $R^1$, $R^2$, $R^3$, $R^4$, $R^5$, $R^6$, Y, Z et m ont les significations indiquées ci-dessus, sur base de leurs propriétés physiques différentes, ensuite, dans une troisième étape, on fait réagir d'abord le diastéréoisomère désiré selon le cas, de formule (IV-a) ou (IV-b), avec un réactif approprié au dédoublement des composés d'oxathianne en présence d'un diluant, ainsi qu'éventuellement en présence d'un agent fixateur d'acide, à des températures comprises entre 0°C et 100°C, et en faisant réagir les n-hydroxyaldéhydes optiquement actifs ainsi formés de formule

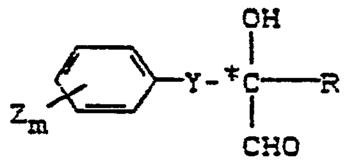
(V)

dans laquelle
R, Z, Y et m ont les significations indiquées ci-dessus, avec un réactif approprié à la réduction d'aldéhydes, en présence d'un diluant, à des températures comprises entre –20°C et +100°C, on fait réagir en outre, dans une quatrième étape, les diols optiquement actifs ainsi obtenus de formule

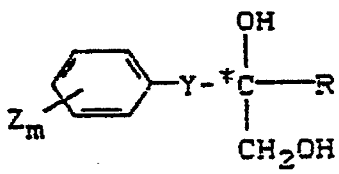
(VI)

dans laquelle
R, Y, Z et m ont les significations indiquées ci-dessus, avec des dérivés d'acide sulfonique de formule

$R^7–SO_2–Hal$ (VII)

dans laquelle
$R^7$ représente un groupe alkyle contenant 1 à 4 atomes de carbone, un groupe halogénalkyle contenant 1 ou 2 atomes de carbone et 1 à 5 atomes d'halogènes, ou un groupe phényle éventuellement substitué par un groupe méthyle, tandis que
Hal représente un atome de chlore ou un atome de brome; en présence d'un diluant et en présence d'un agent fixateur d'aride, à des températures comprises entre 0°C et 100°C, et enfin dans une cinquième étape, on fait réagir les composés optiquement actifs ainsi obtenus de formule

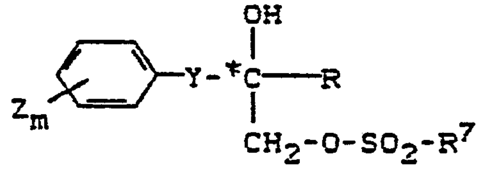
(VIII)

dans laquelle
R, $R^7$, Y, Z et m ont les significations indiquées ci-dessus, avec des sels de triazole de formule

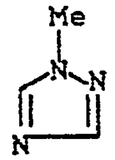

(IX)

dans laquelle
Me représente un atome de sodium ou un atome de potassium, en présence d'un diluant et, éventuellement en présence d'un agent fixateur d'acide, à des températures comprises entre 20°C et 150°C, ou
b) on fait réagir, dans une première étape, des cétones énantiomériquement pures d'oxathianne de formule

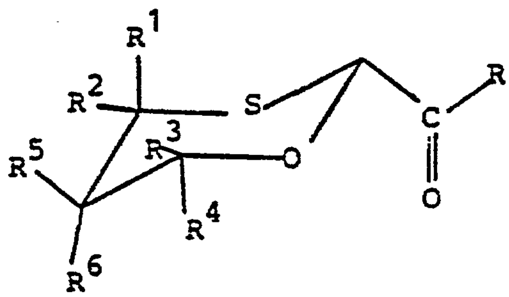

(X)

dans laquelle
R, $R^1$, $R^2$, $R^3$, $R^4$, $R^5$ et $R^6$ ont les significations indiquées ci-dessus, avec des composés organométalliques de formule

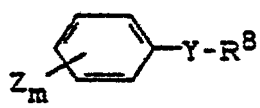

(XI)

dans laquelle
Y, Z et m ont les significations indiquées ci-dessus, tandis que
$R^8$ représente un atome de lithium, un atome de sodium, un atome de potassium, un groupe isopropylate de titane, ou un radical de formule $Me^1X$, dans laquelle $Me^1$ représente un atome de magnésium ou un atome de zinc, tandis que X représente un atome de chlore, un atome de brome ou un atome d'iode, en présence d'un diluant, à des températures comprises entre –78°C et +100°C, ensuite, lors d'une deuxième étape, on fait réagir le diastéréoisomère obtenu préféré de formule

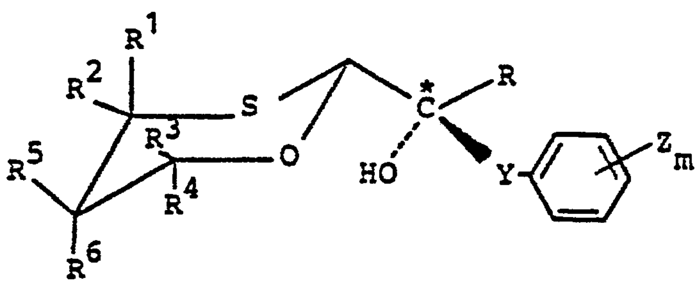

(IV-b)

dans laquelle
R, $R^1$, $R^2$, $R^3$, $R^4$, $R^5$, $R^6$, Y, Z et m ont les significations indiquées ci-dessus, éventuellement après séparation chromatographique antérieure du diastéréoisomère de formule (IV-a) obtenu en quantités moindres, d'abord avec un réactif approprié au dédoublement des composés d'oxathianne, en présence d'un diluant et, éventuellement en présence d'un agent fixateur d'acide, à des températures comprises entre 0°C et 100°C et ensuite, on traite ultérieurement de manière analogue à celle de la variante (a) du procédé, ou
c) dans une première étape, on fait réagir des cétones énantiomériquement pures d'oxathianne de formule

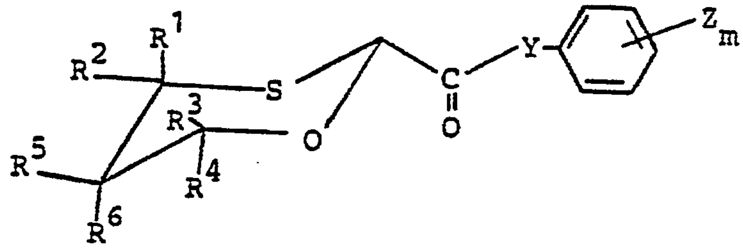

(XII)

dans laquelle
$R^1$, $R^2$, $R^3$, $R^4$, $R^5$, $R^6$, Y, Z et m ont les significations indiquées ci-dessus, avec des composés organométalliques de formule

$$R\text{–}R^8 \quad (XIII)$$

dans laquelle
R et $R^8$ ont les significations indiquées ci-dessus en présence d'un diluant, à des températures comprises entre –78°C et +100°C, ensuite, dans une deuxième étape, on fait réagir le diastéréoisomère obtenu préféré de formule

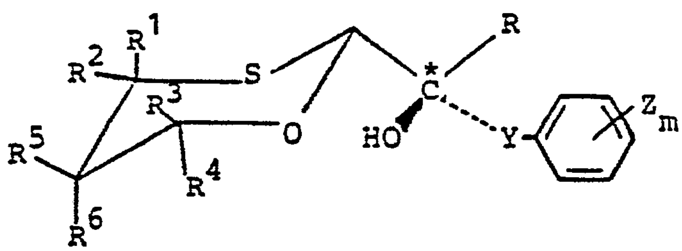

(IV-a)

dans laquelle
R, $R^1$, $R^2$, $R^3$, $R^4$, $R^5$, $R^6$, Y, Z et m ont les significations indiquées ci-dessus, éventuellement après séparation chromatographique antérieure du diastéréoisomère de formule (IV-b) obtenu en quantités moindres, d'abord avec un réactif approprié au dédoublement des composés d'oxathianne, en présence d'un diluant et, éventuellement en présence d'un agent fixateur d'acide, à des températures comprises entre 0°C et 100°C, et ensuite on traite ultérieurement de manière analogue à celle de la variante (a) du procédé.

2. Procédé selon la revendication 1, caractérisé en ce que, comme matière de départ de formule (II), on met en réaction la cétone de formule

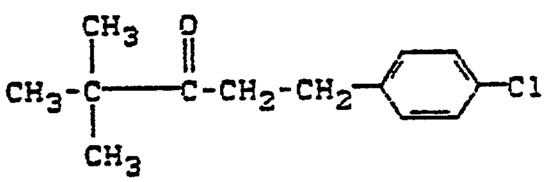

3. Procédé selon la revendication 1, caractérisé en ce que, comme matière de départ de formule (III), on met en réaction l'oxathianne de formule

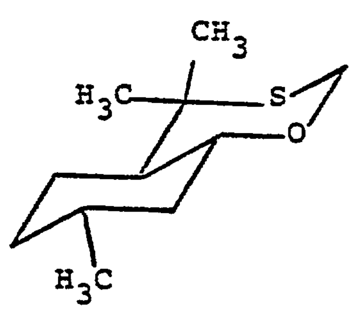

4. Procédé selon la revendication 1, caractérisé en ce que, comme matière de départ de formule (X), on met en réaction la cétone d'oxathianne de formule

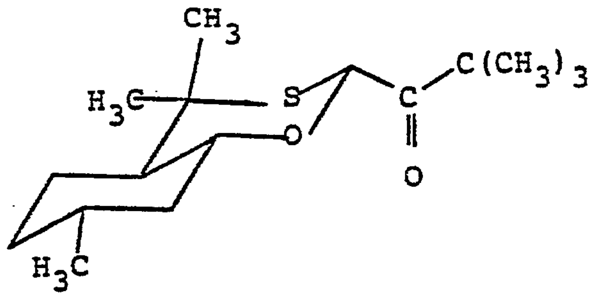

5. Procédé selon la revendication 1, caractérisé en ce que, comme matière de départ de formule (XII), on met en réaction la cétone d'oxathianne de formule